(19) Europäisches Patentamt
European Patent Office
Office européen des brevets

(11) Veröffentlichungsnummer: **0 343 441 B1**

(12) **EUROPÄISCHE PATENTSCHRIFT**

(45) Veröffentlichungstag der Patentschrift: **24.03.93**

(21) Anmeldenummer: **89108533.4**

(22) Anmeldetag: **12.05.89**

(51) Int. Cl.5: **C07C 43/247**, C07C 323/00, C07C 47/55, C07C 205/00, C07C 217/00, C07C 43/225, C07C 69/767, C07C 63/74, C07C 265/00, C07C 69/94, C07C 65/28

# (54) Fluorierte Bis-aryloxy-substituierte Alkene, Verfahren zu deren Herstellung und deren Verwendung.

(30) Priorität: **25.05.88 DE 3817626**

(43) Veröffentlichungstag der Anmeldung:
**29.11.89 Patentblatt 89/48**

(45) Bekanntmachung des Hinweises auf die Patenterteilung:
**24.03.93 Patentblatt 93/12**

(84) Benannte Vertragsstaaten:
**BE CH DE FR GB IT LI NL**

(56) Entgegenhaltungen:

**THE JOURNAL OF ORGANIC CHEMISTRY, Band 45, Nr. 22, 24. Oktober 1980, Seiten 4429-4432, Columbus, Ohio, USA; P. J. CARD et al: "Reaction of Perfluorocycloalkenones with Nucleophiles"**

**CHEMICAL ABSTRACTS, Band 62, Nr. 3, 1. Februar 1986, Spalte 2716f-g, Columbus, Ohio, USA; A. FERRETTI et al: "Reaction of 1,2-dichloroperfluorocycloalkenes with cuprous mercaptides"**

(73) Patentinhaber: **BAYER AG**

**W-5090 Leverkusen 1 Bayerwerk(DE)**

(72) Erfinder: **Negele, Michael, Dr.**
**Wolfskaul 6**
**W-5000 Köln 80(DE)**
Erfinder: **Bielefeldt, Dietmar, Dr.**
**Beuthener Strasse 13**
**W-4030 Ratingen 6(DE)**
Erfinder: **Himmler, Thomas, Dr.**
**Bonhoeffer Strasse 20**
**W-5000 Köln 80(DE)**
Erfinder: **Marhold, Albrecht, Dr.**
**Carl-Duisberg-Strasse 329**
**W-5090 Leverkusen 1(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 343 441 B1

## Beschreibung

Die vorliegende Erfindung betrifft neue fluorierte Bis-aryloxy-substituierte Alkene, ein Verfahren zu deren Herstellung durch Umsetzung von speziell substituierten Phenolen mit speziellen Perhalogenalkenen in basischem Milieu und in Gegenwart von Lösungsmittel und gegebenenfalls anschließender Derivatisierung oder Umderivatisierung und die Verwendung von fluorierten Bis-aryloxysubstituierten Alkenen als Elektroisoliermittel.

Die Substitution beider vinylischer Halogenatome in Dihalogenperfluoralkenen durch Phenole oder durch Phenolate ist bisher durch ein einziges Beispiel belegt: Unter speziellen Bedingungen (-40 ° C, mmol-Maßstab, aufwendige Destillation) konnte aus Natriumphenolat und Octafluorcyclopenten in Ethylenglykoldimethylether das 1,2-Bis-phenoxy-3,3,4,4,5,5-hexafluorcyclopenten-1 in 76 %iger Ausbeute hergestellt werden (siehe J.O.C. 45, 4429 (1980)). Anhand der sonstigen vorliegenden Literatur (z.B. Fluorine in Organic Chemistry, Wiley, 1973) war nicht zu folgern, daß dieser Reaktion ein allgemein gültiges Syntheseprinzip zugrunde liegen könnte. So wurden durch Umsetzung von Phenol oder 2-Naphthol mit Hexafluorcyclobuten in Ether mit äquimolaren Mengen Triethylamin nur Mono-aryloxy-perfluorcyclobutene erhalten (siehe J.A.C.S. 72, 4480 (1950)).

Bei der Reaktion von Phenol mit 1,2-Dichlor-3,3,4,4,5,5-hexafluorcyclopenten-1in Dimethylformamid in Gegenwart äquimolarer Mengen Kaliumhydroxid wurden im Reaktionsprodukt 58,8 % Monosubstitutionsprodukte, 3,4 % geminale Disubstitutionsprodukte und 45,8 % Trisubstitutionsprodukte nachgewiesen (siehe Can. J. Chem. 53, 2302 (1975)). Keines der dort erwähnten geminalen Disubstitutionsprodukte entspricht denen der vorliegenden Erfindung. Aus der gleichen Literaturstelle geht hervor, daß man beim Einsatz von p-Nitrothiophenol anstelle von Phenol das entsprechende Disubstitutionsprodukt (ein Bis-arylthio-substituiertes Alken) in guten Ausbeuten erhält.

Die Umsetzung von o-Aminophenol mit Octafluorcyclopenten in Acetonitril in Gegenwart von Kaliumcarbonat führt zu 2-Heptafluorcyclopentenyl-1-oxyanilin, einem Mono-aryloxy-substituierten Alken. Bei analoger Umsetzung mit o-Aminothiophenol anstelle von o-Aminophenol wird als Zwischenstufe ein Bis-arylthio-substituiertes Cyclopenten erhalten (J.C.S. Perkin Trans. I 763, 1987).

Aus dem Stand der Technik ergibt sich also, daß nur fluorierte Bis-arylthio-substituierte Alkene gut zugänglich sind und fluorierte Bis-aryloxy-substituierte Alkene von anderer Art als die erfindungsgemäßen zumeist nur in Spuren neben großen Menge von mono-und trisubstituierten Produkten zugänglich sind. Es konnte demnach nicht erwartet werden, daß die erfindungsgemäßen fluorierten Bis-aryloxy-substituierten Alkene existent und gut zugänglich sind.

Es wurden nun fluorierte Bis-aryloxy-substituierte Alkene der Formel (I) gefunden,

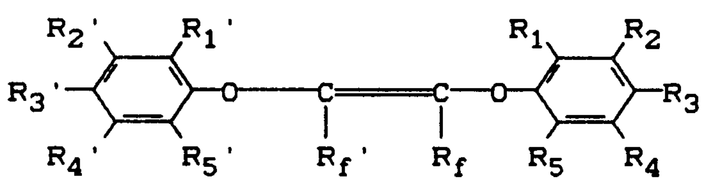 (I),

in der

$R_f$ und $R_f'$ unabhängig voneinander für einen $C_1$- bis $C_6$-Perfluoralkylrest oder $R_f$ und $R_f'$ gemeinsam für einen $C_2$- bis $C_4$-Perfluoralkylenrest und

$R_1$ bis $R_5$ und $R_1'$ bis $R_5'$ unabhängig voneinander für Wasserstoff, für $C_1$- bis $C_{12}$-Alkyl, $C_2$- bis $C_6$-Alkenyl und/oder $C_2$-bis $C_6$-Alkinyl, die gegebenenfalls mit OCN-, $R_6O$-, $H_2N$-, $R_6SO_3$-und/oder $R_6OOC$-Gruppen (mit $R_6$ = Wasserstoff oder gegebenenfalls substituiertem $C_1$- bis $C_6$-Alkyl oder gegebenenfalls substituiertem $C_5$- bis $C_7$-Cycloalkyl) substituiert sind und wobei Alkinylgruppen auch Silyl-Substituenten enthalten können, für $C_1$ - bis $C_4$-Halogenalkyl, für $C_1$- bis $C_4$-Epoxyalkyl, für $C_1$- bis $C_4$-Hydroxyalkyl, für gegebenenfalls substituiertes Phenyl, für $-OR_7$ (mit $R_7$ = Wasserstoff, $C_1$- bis $C_6$-Alkyl, $C_1$- bis $C_4$-Hydroxyalkyl, $C_1$-bis $C_4$-Epoxyalkyl, $-(-CH_2)_m-[-O-(-CH_2)_m-]-_nOH$ mit m = 1 bis 4 und n = 1 bis 8, gegebenenfalls substituiertem Phenyl oder gegebenenfalls substituiertem Benzyl), für Halogen, für $NO_2$, für $NH_2$, für NCO, für CN, für $SO_3X$ (mit X = Wasserstoff, Natrium oder Kalium), für S-$C_1$- bis $C_6$-Alkyl, für $SO_2$Halogen, für

gegebenenfalls substituiertes $C_5$- bis $C_7$-Cycloalkyl, für Carbonyl-$R_8$ (mit $R_8$ = Wasserstoff, gegebenenfalls substituiertem $C_1$- bis $C_6$-Alkyl, gegebenenfalls substituiertem $C_5$- bis $C_7$-Cycloalkyl, Halogen, gegebenenfalls substituiertem $C_1$-bis $C_6$-Alkoxy, gegebenenfalls substituiertem $C_5$- bis $C_7$-Cycloalkoxy oder OY mit Y = Wasserstoff, Natrium oder Kalium) oder für

$$\overset{|}{(R_9CO)N}(R_{10})$$

(mit $R_9$ = $C_1$- bis $C_6$-Alkyl, $C_1$-bis $C_4$-Halogenalkyl, gegebenenfalls substituiertem Phenyl oder Wasserstoff und mit $R_{10}$ = Wasserstoff oder $C_1$- bis $C_4$-Alkyl) stehen,

wobei die Verbindung ausgenommen ist, bei der $R_1$ bis $R_5$ und $R_1'$ bis $R_5'$ für Wasserstoff und $R_f$ und $R_f'$ gemeinsam für $-(-CF_2-)-_3$ stehen.

Wenn $R_f$ und $R_f'$ gemeinsam für einen Perfluoralkylenrest stehen, bilden $R_f$, $R_f'$ und die dazwischenliegenden beiden C-Atome eine 4- bis 6-gliedrige perfluorcycloalkylgruppe.

In $C_2$- bis $C_6$-Alkenyl- und -Alkinylresten können eine oder mehrere Mehrfachbindungen enthalten sein, die innenständig oder endständig angeordnet sein können. Vorzugsweise ist nur eine Mehrfachbindung enthalten, die vorzugsweise endständig angeordnet ist.

Bei $C_1$- bis $C_4$-Halogenalkylresten kann es sich um Reste mit einem oder mehreren, gleichen oder verschiedenen Halogenatomen handeln. Als Halogenatome kommen Fluor, Chlor, Brom und/oder Iod in Frage, bevorzugt sind Fluor, Chlor und/oder Brom, insbesondere Fluor und/oder Chlor, Daz bzw. die Halogenatome können innenständig oder endständig angeordnet sein.

$C_1$- bis $C_4$-Epoxy- und $C_1$- bis $C_4$-Hydroxyalkylreste (als $R_1$ bis $R_5$, $R_1'$ bis $R_5'$ und/oder als $R_6$) enthalten vorzugsweise nur eine Epoxy- oder eine Hydroxygruppe. Diese befindet sich vorzugsweise in endständiger Anordnung.

Sofern Phenylreste (als $R_1$ bis $R_5$, $R_1'$ bis $R_5'$ und/oder als $R_6$) substituiert sein können kommen als Substituenten beispielsweise $NO_2$, $NH_2$, NCO, COOH, CHO, OH, $SO_3H$, F, Cl und/oder Br in Frage.

Sofern Benzylreste substituiert sein können kommen als Substituenten die gleichen in Frage wie bei substituierten Phenylresten.

Bei Halogenresten kann es sich um Fluor-, Chlor-, Brom-oder Iodatome handeln. Bevorzugt sind Fluor-, Chlor- und Bromatome, insbesondere Fluor- und Chloratome.

$SO_2$-Halogen-Reste können z.B. als Halogen Fluor, Chlor oder Brom enthalten. Bevorzugt sind Fluor und Chlor.

Sofern $C_5$- bis $C_7$-Cycloalkylreste substituiert sein können, kommen als Substituenten beispielsweise $C_1$- bis $C_6$-Alkyl, $C_2$- bis $C_6$-Alkenyl, $C_1$- bis $C_4$-Haloalkyl, gegebenenfalls substituiertes Phenyl, Halogen, $NH_2$, COOH oder OH in Frage.

Die Reste $R_1$ bis $R_5$ können gleich oder voneinander ganz oder teilweise verschieden sein. Unabhängig von der Bedeutung von $R_1$ bis $R_5$ können auch die Reste $R_1'$ bis $R_5'$ gleich oder voneinander ganz oder teilweise verschieden sein.

Grundsätzlich bevorzugt sind $R_f$ und $R_f'$ gleich und stehen für $CF_3$ (wobei die beiden $CF_3$-Gruppen im Hinblick auf die zentrale C=C-Doppelbindung in Formel (I) in cis-oder trans-Konfiguration vorliegen können) oder $R_f$ und $R_f'$ bilden gemeinsam eine $-CF_2-CF_2-$ oder eine $-CF_2-CF_2-CF_2$-Gruppe.

Grundsätzlich bevorzugt sind jeweils $R_1$ und $R_1'$ und unabhängig davon jeweils $R_5$ und $R_5'$ gleich und stehen für Wasserstoff, $C_1$- bis $C_4$-Alkyl, $C_1$- bis $C_4$-Halogenalkyl, $NO_2$, NCO, Fluor oder Chlor. Besonders bevorzugt sind $R_1$, $R_1'$, $R_5$ und $R_5'$ gleich und stehen für Wasserstoff oder Chlor, insbesondere für Wasserstoff oder $R_1$ und $R_1'$ stehen für Wasserstoff und $R_5$ und $R_5'$ für $CF_3$.

Grundsätzlich bevorzugt sind jeweils $R_2$ und $R_2'$ und unabhängig davon jeweils $R_4$ und $R_4'$ gleich und stehen für Wasserstoff, $C_1$- bis $C_4$-Alkyl, COOH, Fluor, Chlor oder $C_1$- bis $C_4$-Halogenalkyl. Besonders bevorzugt sind $R_2$, $R_2'$, $R_4$ und $R_4'$ gleich und stehen für Wasserstoff, Chlor oder $CF_3$ oder $R_2$ und $R_2'$ stehen für Wasserstoff und $R_4$ und $R_4'$ für $CH_3$.

Grundsätzlich bevorzugt stehen $R_3$ und $R_3'$ unabhängig voneinander für Wasserstoff, für $C_1$- bis $C_{12}$-Alkyl, $C_2$-bis $C_6$-Alkenyl und/oder $C_2$- bis $C_6$-Alkinyl, die gegebenenfalls mit OCN-, $R_6O$-, $H_2N$-, $R_6SO_3$- und/oder $R_6OOC$-Gruppen (mit $R_6$ = Wasserstoff oder gegebenenfalls substituiertem $C_1$- bis $C_6$-Alkyl oder gegebenenfalls substituiertem $C_5$- bis $C_7$-Cycloalkyl) substituiert sind und wobei Alkinylgruppen auch Silyl-Substituenten, insbesondere Trimethylsilyl-Substituenten, enthalten können, für $C_1$- bis $C_4$-Halogenalkyl, für $C_1$-bis $C_4$-Epoxyalkyl, für $C_1$- bis $C_4$-Hydroxyalkyl, für gegebenenfalls substituiertes Phenyl, für-$OR_7$ (mit $R_7$

= Wasserstoff, $C_1$- bis $C_6$-Alkyl, $C_1$-bis $C_4$-Hydroxyalkyl, $C_1$- bis $C_4$-Epoxyalkyl, $-(-CH_2)_m-[-O-(-CH_2)_m-]-_nOH$ mit m = 1 bis 4 und n = 1 bis 8, gegebenenfalls substituiertem Phenyl oder gegebenenfalls substituiertem Benzyl), für Halogen, für $NO_2$, für $NH_2$, für NCO, für CN, für $SO_3X$ (mit X = Wasserstoff, Natrium oder Kalium), für S-$C_1$-bis $C_6$-Alkyl, für $SO_2$-Halogen, für gegebenenfalls substituiertes $C_5$- bis $C_7$-Cycloalkyl,für Carbonyl-$R_8$ (mit $R_8$ = Wasserstoff, gegebenenfalls substituiertem $C_1$- bis $C_6$-Alkyl, gegebenenfalls substituiertem $C_5$- bis $C_7$-Cycloalkyl, Halogen, gegebenenfalls substituiertem $C_1$-bis $C_6$-Alkoxy, gegebenenfalls substituiertem $C_5$- bis $C_7$-Cycloalkoxy oder OY mit Y = Wasserstoff, Natrium oder Kalium) oder für

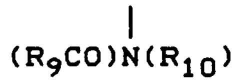

(mit $R_9$ = $C_1$- bis $C_6$-Alkyl, $C_1$- bis $C_4$-Halogenalkyl, gegebenenfalls substituiertem Phenyl oder Wasserstoff und mit $R_{10}$ = Wasserstoff oder $C_1$- bis $C_4$-Alkyl).

Grundsätzlich besonders bevorzugt sind $R_3$ und $R_3'$ gleich und stehen für Wasserstoff, $C_1$- bis $C_{12}$-Alkyl, Ethenyl, Ethinyl, $CF_3$, $C_2$- bis $C_3$-Epoxyalkyl, $C_1$- bis $C_3$-Hydroxyalkyl, OH, O-$C_1$- bis $C_3$-Alkyl, O-Benzyl, Fluor, Chlor, Brom, $NO_2$, $NH_2$, NCO, S-$C_1$- bis $C_3$-Alkyl, COOH, CHO, CO-$C_1$- bis $C_3$-Alkyl, COCl oder CO-O-$C_1$- bis $C_3$-Alkyl.

Speziell bevorzugte Einzelverbindungen der Formel (I) sind in der Tabelle 1 zusammengestellt.

**Tabelle 1**

| $R_f$ $R_f'$ | sonstige Substituenten (nichtgenannte = H) |
|---|---|
| $-(-CF_2-)-_2$ | $R_3$ = $R_3'$ = $-NO_2$ |
| $-(-CF_2-)-_2$ | $R_3$ = $R_3'$ = $-NH_2$ |
| $-(-CF_2-)-_2$ | $R_3$ = $R_3'$ = -NCO |
| $-(-CF_2-)-_2$ | $R_3$ = $R_3'$ = -CN |
| $-(-CF_2-)-_2$ | $R_3$ = $R_3'$ = -COOH |
| $-(-CF_2-)-_2$ | $R_3$ = $R_3'$ = $-CO-OCH_3$ |
| $-(-CF_2-)-_2$ | $R_3$ = $R_3'$ = -CO-O-(L)-Menthyl |
| $-(-CF_2-)-_2$ | $R_3$ = $R_3'$ = -OH |
| $-(-CF_2-)-_2$ | $R_3$ = $R_3'$ = -CHO |
| $-(-CF_2-)-_2$ | $R_3$ = $R_3'$ = $-OCH_2$ 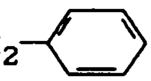 |
| $-(-CF_2-)-_2$ | $R_3$ = $R_3'$ = $-NH-COCH_3$ |
| $-(-CF_2-)-_2$ | $R_3$ = $R_3'$ = -Cl |
| $-(-CF_2-)-_2$ | $R_3$ = $R_3'$ = -Br |
| $-(-CF_2-)-_2$ | $R_1$ = $R_1'$ = $-CF_3$; $R_3$ = $R_3'$ = $-NO_2$ |
| $-(-CF_2-)-_2$ | $R_1$ = $R_1'$ = $-CF_3$; $R_3$ = $R_3'$ = $-NH_2$ |
| $-(-CF_2-)-_2$ | $R_1$ = $R_1'$ = $-CF_3$; $R_3$ = $R_3'$ = -NCO |
| $-(-CF_2-)-_3$ | $R_3$ = $R_3'$ = $-CH_3$ |
| $-(-CF_2-)-_3$ | $R_3$ = $R_3'$ = $-NO_2$ |
| $-(-CF_2-)-_3$ | $R_3$ = $R_3'$ = $-C_9H_{19}$ |
| $-(-CF_2-)-_3$ | $R_2$ = $R_2'$ = $R_3$ = $R_3'$ = $-CH_3$ |
| $-(-CF_2-)-_3$ | $R_3$ = $R_3'$ = $-NH_2$ |
| $-(-CF_2-)-_3$ | $R_3$ = $R_3'$ = $-OCH_3$ |
| $-(-CF_2-)-_3$ | $R_3$ = $R_3'$ = -NCO |
| $-(-CF_2-)-_3$ | $R_3$ = $R_3'$ = $-OCH_2$ 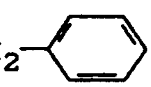 |
| $-(-CF_2-)-_3$ | $R_1$ = $R_1'$= $R_3$ = $R_3'$= Cl; $R_2$ = $R_2'$ = $NO_2$ |

Tabelle 1 (Fortsetzung)

| $R_f$ $R_f$' | sonstige Substituenten (nichtgenannte = H) |
|---|---|
| $-(-CF_2-)-_3$ | $R_3$ = $R_3$' = $-CO-OCH_3$ |
| $-(-CF_2-)-_3$ | $R_3$ = $R_3$' = -COOH |
| $-(-CF_2-)-_3$ | $R_3$ = $R_3$' = -COCl |
| $-(-CF_2-)-_3$ | $R_3$ = $R_3$' = -OH |
| $-(-CF_2-)-_3$ | $R_3$ = $R_3$' = -Cl |
| $-(-CF_2-)-_3$ | $R_3$ = $R_3$' = -CHO |
| $-(-CF_2-)-_3$ | $R_3$ = $R_3$' = -Br |
| $-(-CF_2-)-_3$ | $R_3$ = $R_3$' = $-CH_2OH$ |
| $-(-CF_2-)-_3$ | $R_3$ = $R_3$' = -F |
| $-(-CF_2-)-_3$ | $R_1$ = $R_1$' = $-CF_3$; $R_3$ = $R_3$' = $-NO_2$ |
| $-(-CF_2-)-_3$ | $R_2$ = $R_2$' = $R_3$ = $R_3$' = -COOH |
| $-(-CF_2-)-_3$ | $R_3$ = $R_3$' = -COONa |
| $-(-CF_2-)-_3$ | $R_3$ = $R_3$' = $-SCH_3$ |
| $-(-CF_2-)-_3$ | $R_2$ = $R_2$' = $R_4$ = $R_4$' = $-CF_3$ |
| $-(-CF_2-)-_3$ | $R_3$ = $CH_3$; $R_3$' = $-NO_2$ |
| $-(-CF_2-)-_3$ | $R_1$ bis $R_5$ = $R_1$' bis $R_5$' = -Cl |
| $-(-CF_2-)-_3$ | $R_3$ = $R_3$' = Ethinyl |
| $-(-CF_2-)-_3$ | $R_3$ = $R_3$' = Ethenyl |
| $-(-CF_2-)-_3$ | $R_3$ = $R_3$' = 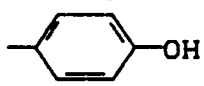 |
| $-(-CF_2-)-_3$ | $R_3$ = $R_3$' = Ethylenoxy |
| $-(-CF_2-)-_3$ | $R_2$ = $R_2$' = $R_3$ = $R_3$' = $-CF_3$ |
| $-(-CF_2-)-_3$ | $R_1$ = $R_1$' = $-CF_3$ |
| $-(-CF_2-)-_3$ | $R_3$ = $R_3$' = 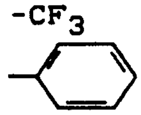 |
| $-(-CF_2-)-_3$ | $R_3$ = $R_3$' = 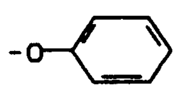 |

<u>Tabelle 1</u> (Fortsetzung)

| $R_f$ $R_f$' | sonstige Substituenten (nichtgenannte = H) |
|---|---|
| $-(-CF_2-)-_3$ | $R_3$ = $R_3$' = Methylethenyl |
| $-(-CF_2-)-_3$ | $R_3$ = $R_3$' = $-CO-CH_2-$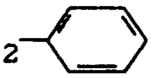 |
| $-(-CF_2-)-_3$ | $R_3$ = $R_3$' = 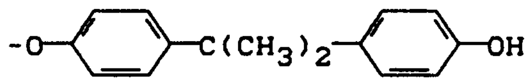 |
| $-(-CF_2-)-_3$ | $R_3$ = $R_3$' = $-CO-OC_5H_{11}$ |
| $-(-CF_2-)-_3$ | $R_3$ = $R_3$' = $-NH-COOCH_3$ |
| $-(-CF_2-)-_3$ | $R_3$ = $R_3$' = -CO-O-(L)-Menthyl |
| $-(-CF_2-)-_3$ | $R_3$ = $R_3$' = -CN |
| $-(-CF_2-)-_3$ | $R_3$ = $R_3$' = -Propylenoxy |
| $-(-CF_2-)-_3$ | $R_3$ = $R_3$' = $-OCH_2CH_2OH$ |
| $-(-CF_2-)-_3$ | $R_3$ = $R_3$' = 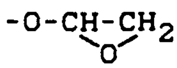 |
| $-(-CF_2-)-_3$ | $R_3$ = $R_3$' = $-SO_3H$ |
| $-(-CF_2-)-_3$ | $R_3$ = $R_3$' = $-SO_3Na$ |
| $-(-CF_2-)-_3$ | $R_3$ = $R_3$' = $-SO_2Cl$ |
| $-(-CF_2-)-_3$ | $R_3$ = $R_3$' = $-C(CH_3)=CH_2$ |
| $-(-CF_2-)-_3$ | $R_3$ = $R_3$' = $-CH=CH-CO-OC_2H_5$ |
| $-(-CF_2-)-_3$ | $R_3$ = $R_3$' = $-CH=CH-CO-NH_2$ |
| $-(-CF_2-)-_3$ | $R_3$ = $R_3$' = -CH=CH-CN |
| $-(-CF_2-)-_3$ | $R_3$ = $R_3$' = -CH=CH-COOH |
| $-(-CF_2-)-_3$ | $R_3$ = $R_3$' = $-C\equiv C-C(CH_3)_2OH$ |
| $-(-CF_2-)-_3$ | $R_3$ = $R_3$' = $-C\equiv C-Si-(-CH_3)_3$ |
| $-(-CF_2-)-_3$ | $R_3$ = $R_3$' = $-C\equiv C-$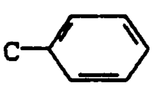 |

<u>Tabelle 1</u> (Fortsetzung)

| $R_f$ $R_f$' | sonstige Substituenten (nichtgenannte = H) |
|---|---|
| $-(-CF_2-)-_3$ | $R_3$ = $-CH_3$; $R_3$' = $-NH_2$ |
| $-(-CF_2-)-_3$ | $R_3$ = -COOH; $R_3$' = $-NH_2$ |
| $-(-CF_2-)-_3$ | $R_3$ = -CN; $R_3$' = $-CO-OCH_3$ |
| $-(-CF_2-)-_3$ | $R_3$ = -CN; $R_3$' = -CO-O-(L)-Menthyl |
| $-(-CF_2-)-_3$ | $R_3$ = -C≡CH; $R_3$' = $-CO-OCH_3$ |
| $-(-CF_2-)-_3$ | $R_3$ = -C≡CH; $R_3$' = -CO-O-(L)-Menthyl |
| $-(-CF_2-)-_3$ | $R_2$ = $R_2$' = $-NH_2$; $R_3$ = $R_3$' = -OH |
| $-(-CF_2-)-_3$ | $R_1$ = $R_1$' = $R_3$ = $R_3$' = $-NO_2$ |
| $-(-CF_2-)-_3$ | $R_1$ = $R_1$' = $R_3$ = $R_3$' = $-NH_2$ |
| $-(-CF_2-)-_3$ | $R_1$ = $R_1$' = $R_3$ = $R_3$' = -NCO |
| $-(-CF_2-)-_3$ | $R_1$ = $R_1$' = $R_3$ = $R_3$' = $R_4$ = $R_4$' = Cl |
| $-(-CF_2-)-_3$ | $R_1$ = $R_1$' = $R_3$ = $R_3$' = Cl |
| $-(-CF_2-)-_3$ | $R_2$ = $R_2$' = $NO_2$; $R_3$ = $R_3$' = $-OCH_3$ |
| $-(-CF_2-)-_3$ | $R_1$ = $R_1$' = $CF_3$; $R_3$ = $R_3$' = $-NH_2$ |
| $-(-CF_2-)-_3$ | $R_1$ = $R_1$' = $CF_3$; $R_3$ = $R_3$' = -NCO |
| $-(-CF_2-)-_3$ | $R_2$ = $R_2$' = $R_3$ = $R_3$' = -COOH |
| $-(-CF_2-)-_3$ | $R_2$ = $R_2$' = $-CO-OCH_3$ |
| $-(-CF_2-)-_3$ | $R_2$ = $R_2$' = -COOH |
| $-(-CF_2-)-_3$ | $R_2$ = $R_2$' = -COCl |
| $-(-CF_2-)-_3$ | $R_2$ = $R_2$' = $-NO_2$ |
| $-(-CF_2-)-_3$ | $R_2$ = $R_2$' = $-NH_2$ |
| $-(-CF_2-)-_3$ | $R_2$ = $R_2$' = -NCO |
| $-(-CF_2-)-_3$ | $R_3$ = $R_3$' = 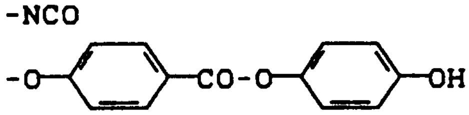 |
| $-(-CF_2-)-_3$ | $R_3$ = -Br; $R_3$' = -CN |
| $-(-CF_2-)-_3$ | $R_3$ = -C≡CH; $R_3$' = -CN |

Tabelle 1 (Fortsetzung)

| $R_f$ | $R_f'$ | sonstige Substituenten (nichtgenannte = H) |
|---|---|---|
| $-(-CF_2-)-_3$ | | $R_3$ = $-CH = CH-CO-OC_2H_5$; $R_3'$ = -CN |
| $-(-CF_2-)-_3$ | | $R_3$ = -Br; $R_3'$ = $-CO-OCH_3$ |
| $-(-CF_2-)-_3$ | | $R_3$ = $-CH=CH-O-OC_2H_5$; $R_3'$ = $-CO-OCH_3$ |
| $-(-CF_2-)-_3$ | | $R_3$ = CN; $R_3'$ = Cyclohexyl |
| $-(-CF_2-)-_4$ | | $R_3$ = $R_3'$ = $-NO_2$ |
| $-(-CF_2-)-_4$ | | $R_3$ = $R_3'$ = $-NH_2$ |
| $-(-CF_2-)-_4$ | | $R_3$ = $R_3'$ = -NCO |
| $-(-CF_2-)-_4$ | | $R_3$ = $R_3'$ = $-CO-OCH_3$ |
| $-(-CF_2-)-_4$ | | $R_3$ = $R_3'$ = -CO-O-(L)-Menthyl |
| $-(-CF_2-)-_4$ | | $R_3$ = $R_3'$ = -COOH |
| $-(-CF_2-)-_4$ | | $R_3$ = $R_3'$ = -CHO |
| $-(-CF_2-)-_4$ | | $R_3$ = $R_3'$ = -CN |
| $-(-CF_2-)-_4$ | | $R_3$ = $R_3'$ = $-NH-COCH_3$ |
| $-(-CF_2-)-_4$ | | $R_3$ = $R_3'$ = -OH |
| $CF_3$ | $CF_3$ | $R_3$ = $R_3'$ = $-NO_2$ |
| $CF_3$ | $CF_3$ | $R_3$ = $R_3'$ = $-NH_2$ |
| $CF_3$ | $CF_3$ | $R_3$ = $R_3'$ = -NCO |
| $CF_3$ | $CF_3$ | $R_3$ = $R_3'$ = $-CO-OCH_3$ |
| $CF_3$ | $CF_3$ | $R_3$ = $R_3'$ = -COOH |
| $CF_3$ | $CF_3$ | $R_3$ = $R_3'$ = -OH |
| $CF_3$ | $CF_3$ | $R_3$ = $R_3'$ = $-O-CH_2$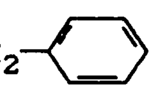 |
| $CF_3$ | $CF_3$ | $R_3$ = $R_3'$ = -CN |
| $CF_3$ | $CF_3$ | $R_3$ = $R_3'$ = -CO-O-(L)-Menthyl |

**Tabelle 1** (Fortsetzung)

| $R_f$ | $R_f'$ | sonstige Substituenten (nichtgenannte = H) |
|---|---|---|
| $CF_3$ | $CF_3$ | $R_3$ = $R_3'$ = -CHO |
| $CF_3$ | $CF_3$ | $R_3$ = $R_3'$ = -C≡CH |
| $CF_3$ | $CF_3$ | $R_3$ = $R_3'$ = $-CH=CH-CO-OC_2H_5$ |
| $CF_3$ | $CF_3$ | $R_2$ = $R_2'$ = $R_4$ = $R_4'$ = $CF_3$ |

Speziell bevorzugt sind auch Verbindungen der Formel (I), bei denen $R_f$ und $R_f'$ jeweils für $CF_3$ stehen oder $R_f$ und $R_f'$ gemeinsam eine $-CF_2-CF_2-$ oder eine $-CF_2-CF_2-CF_2-$Gruppe bedeuten, $R_1$, $R_2$, $R_4$, $R_5$, $R_1'$, $R_2'$, $R_4'$ und $R_5'$ für Wasserstoff stehen und $R_3$ und $R_3'$ gleich sind und eine in Anspruch 3 angegebene, von Wasserstoff verschiedenen Bedeutung haben.

Das erfindungsgemäße Verfahren zur Herstellung von fluorierten Bis-aryloxy-substituierten Alkenen der Formel (I) ist dadurch gekennzeichnet, daß man ein Phenol und/oder Phenolat der Formel (II)

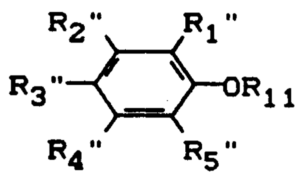 (II),

in der

$R_1''$ bis $R_5''$ unabhängig voneinander für Wasserstoff, für $C_1$-bis $C_{12}$-Alkyl, $C_2$- bis $C_6$-Alkenyl und/oder $C_2$- bis $C_6$-Alkinyl, die gegebenenfalls mit $R_6'O$-, $(R_6')_2N$-, $R_6'SO_3$- und/oder $R_6'OOC$-Gruppen (mit $R_6'$ = gegebenenfalls substituiertem $C_1$- bis $C_6$-Alkyl oder gegebenenfalls substituiertem $C_5$- bis $C_7$-Cycloalkyl) substituiert sind und wobei Alkinylgruppen auch Silyl-Substituenten enthalten können, für $C_1$-bis $C_4$-Halogenalkyl, für gegebenenfalls substituiertes Phenyl, für $OR_7'$ (mit $R_7'$ = $C_1$-bis $C_6$-Alkyl, gegebenenfalls substituiertem Phenyl oder gegebenenfalls substituiertem Benzyl), für Halogen, für $NO_2$, für $NH_2$ mit nicht aciden H-Atomen, für CN, für $SO_3X'$ (mit $X'$ = Natrium oder Kalium) für S-$C_1$- bis $C_6$-Alkyl, für gegebenenfalls substituiertes $C_5$- bis $C_7$-Cycloalkyl,für Carbonyl-$R_8'$ (mit $R_8'$ = gegebenenfalls substituiertem $C_1$-bis $C_6$-Alkyl, $C_1$-bis $C_6$-Alkoxy, $C_5$- bis $C_7$-Cycloalkyl oder $C_5$-bis $C_7$-Cycloalkoxy oder $OY'$ mit $Y'$ = Natrium oder Kalium) oder für

|
$(R_9CO)N(R_{10})$

(mit $R_9$ = $C_1$- bis $C_6$-Alkyl, $C_1$-bis $C_4$-Halogenalkyl, gegebenenfalls substituiertem Phenyl oder Wasserstoff und mit $R_{10}$ = Wasserstoff oder $C_1$-bis $C_4$-Alkyl) und

$R_{11}$ für Wasserstoff, ein Alkalimetall oder einen Tri-$C_1$-$C_4$-alkylsilylrest stehen

mit einem Perhalogenalken der Formel (III)

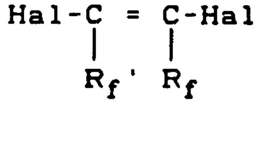 (III),

in der

$R_f$ und $R_f'$ die bei Formel (I) angegebene Bedeutung haben und

Hal für Fluor, Chlor, Brom und/oder Iod stehen,

in basischem Milieu und in Gegenwart eines Lösungsmittels umsetzt und gegebenenfalls anschließend eine Derivatisierung und/oder Umderivatisierung durchführt, wobei die Umsetzung von unsubstituiertem Phenol und unsubstituierten Phenolaten (Formel (II), $R_1''$ bis $R_5''$ = H, $R_{11}$ = H oder Alkalimetall) mit Octafluorcyclopenten (Formel (III)), $R_f$ und $R_f'$ = gemeinsam $(CF_2)_3$, Hal = F) ausgenommen ist.

Soweit in Formel (II) $R_1''$ bis $R_5''$ die gleiche Bedeutung haben wie $R_1$ bis $R_5$ in Formel (I) stehen $R_1''$ bis $R_5''$ vorzugsweise für das, was bei der entsprechenden Offenbarung zu $R_1$ bis $R_5$ als bevorzugt angegeben ist. $R_{11}$ steht vorzugsweise für Natrium oder Kalium.

In Formel (III) steht Hal vorzugsweise für Fluor, Chlor und/oder Brom, insbesondere für Fluor und/oder Chlor. $R_f$ und $R_f'$ bedeutet vorzugsweise das, was bei Formel (I) als bevorzugt beschrieben ist.

Das für das erfindungsgemäße Verfahren erforderliche basische Milieu läßt sich auf verschiedene Weise realisieren. Beim Einsatz von Verbindungen der Formel (II) mit $R_{11}$ = Alkalimetall (d.h. Phenolaten, die basisch sind) sind weitere Maßnahmen zur Realisierung eines basischen Milieus nicht unbedingt erforderlich. Beim Einsatz von Verbindungen der Formel (II) mit $R_{11}$ = Wasserstoff (d.h. Phenolen) oder mit $R_{11}$ = Tri-$C_1$-$C_4$-alkylsilyl ist es erforderlich, basisch wirkende Stoffe hinzuzufügen. Im allgemeinen setzt man Basen etwa in der stöchiometrisch erforderlichen Menge ein, beispielsweise 1,7 bis 2,3 Mol Base pro Mol der Verbindung der Formel (III). Sind in der eingesetzten Verbindung der Formel (II) acide Wasserstoffatome vorhanden, setzt man zweckmäßigerweise zusätzlich mindestens die zu deren Neutralisation erforderliche Menge Base zu, da sonst unerwünschte Nebenreaktionen ablaufen können.

Als Base kommen die verschiedensten anorganischen und organischen alkalisch reagierenden Substanzen in Frage, beispielsweise Oxide, Hydroxide, Carbonate und Hydrogencarbonate von Alkali- und Erdalkalimetallen und Amine, insbesondere tertiäre Amine. Bevorzugt sind Alkalihydroxide, Alkalicarbonate, Triethylamin und Pyridin. Besonders bevorzugt sind Natrimhydroxid und Triethylamin.

Sollen Phenolate eingesetzt werden (Formel (II), $R_{11}$ = Alkalimetall) so können diese aus den entsprechenden Phenolen auf verschiedene Weise erhalten werden. Beispielsweise kann man die Phenole mit Alkalimetallen, Alkalihydroxiden, Alkalihydriden oder Alkalialkoholaten umsetzen. Bevorzugt sind dabei Umsetzungen mit Natrium, Natriumhydroxid, Natriumhydrid, Natriummethylat oder Natriumethylat.

Als Lösungsmittel kommen beispielsweise inerte organische Lösungsmittel in Frage, in Sonderfällen in Kombination mit Wasser. Geeignete Lösungsmittel sind z.B. Ether, Kohlenwasserstoffe, chlorierte Kohlenwasserstoffe, Nitrile, Amide, Sulfone und organische Carbonate. Als Beispiele seien genannt: Methyl-tert.-butylether, Diethylether, Tetrahydrofuran, Dioxan, Methylenchlorid, Trichlorethylen, Chloroform, Tetrachlorkohlenstoff, Benzol, toluol, Xylol, Acetonitril, Dimethylformamid, Tetramethylensulfon, Tetramethylharnstoff, Hexamethylphosphorsäuretriamid und Propylencarbonat. Bevorzugte Lösungsmittel sind Diethylether, Methylenchlorid, Acetonitril und Dimethylformamid.

Das erfindungsgemäße Verfahren kann beispielsweise bei Temperaturen im Bereich von -78 bis +40 ° C durchgeführt werden. Vorzugsweise arbeitet man bei 0 bis 25 ° C. Wenn man unter erhöhtem Druck arbeitet, kann man die Reaktionstemperatur auch wesentlich über 40 ° C hinaus steigern, beispielsweise bis zu 150 ° C.

Das Molverhältnis der jeweils eingesetzten Verbindung der Formel (II) zu der jeweils eingesetzten Verbindung der Formel (III) kann beispielsweise 1,9:1 bis 3:1 betragen. Vorzugsweise werden stöchiometrische Mengen eingesetzt, also 2 Mole der Verbindung der Formel (II) pro Mol Verbindung der Formel (III). Man kann das erfindungsgemäße Verfahren auch so durchführen, daß man in einer ersten Stufe nur ein Hal aus der Verbindung der Formel (III) durch einen Aryloxyrest entsprechend dem Phenol(at) der Formel (II) ersetzt und in einer zweiten Stufe das zweite Hal der Verbindung der Formel (III) durch einen gleichen oder anderen Aryloxyrest wie in der ersten Stufe eingeführt, ersetzt. Bei der zweistufigen Verfahrensvariante kann man beispielsweise so verfahren, daß man in die erste Stufe nur maximal 1 Mol einer Verbindung der Formel (II) pro Mol einer Verbindung der Formel (III) einsetzt und in die zweite Stufe mindestens ein weiteres Mol der gleichen oder einer anderen Verbindung der Formel (III). Auch durch Zugabe der erforderlichen Basenmenge in zwei etwa gleichen Portionen, kann man eine 2-stufige Verfahrensweise realisieren.

Nicht alle fluorierten Bis-aryloxy-substituierten Alkene der Formel (I) sind direkt aus einem entsprechenden Phenol oder Phenolat der Formel (II) und einem entsprechenden Perhalogenalken der Formel (III) zugänglich. Verbindungen der Formel (I), die beispielsweise COOH-, $NH_2$-mit aciden H-Atomen, NCO-, OH- und/oder $SO_3H$-Gruppen enthalten, müssen unter Umständen nach dem Aufbau des Bis-aryloxy-alken-Gerüstes derivatisiert oder umderivatisiert werden. Beispiele hierfür sind die Einführung von $SO_3H$-Gruppen durch Umsetzung mit Schwefelsäure, die Reduktion von $NO_2$- zu $NH_2$-Gruppen, die Etherspaltung von

Alkoxy- zu OH-Gruppen, die Oxidation von $CH_3$- zu COOH-Gruppen und das Ansäuern von $SO_3Na$-, COONa-, $SO_3NR_3H$-oder $COO(NR_3H)$-Gruppen zur Bildung von $SO_3H$- oder COOH-Gruppen, die Verseifung von Carbonsäureestern oder Nitrilen zu Carbonsäuren, die Phosgenierung von $NH_2$- zu NCO-Gruppen und die Oxidation von S-Alkyl zu $SO_2Cl$-Gruppen mit elementarem Chlor in Wasser.

Verbindungen der Formel (I), die prinzipiell durch direkte Synthese aus einer Verbindung der Formel (II) und einer Verbindung der Formel (III) zugänglich sind, können auch durch Derivatisierung oder Umderivatisierung erhalten werden. Beispielsweise kann man 1,2-Bis-(4-carboxyphenoxy)-3,3,4,4,5,5-hexafluorcyclopenten-1 herstellen entweder indem man 4-Hydroxybenzoesäuremethylester mit 1,2-Dichlor-3,3,4,4,5,5-hexafluorcyclopenten-1 oder Octafluorcyclopenten umsetzt und anschließend verseift oder indem man Na-4-methylphenolat mit Octafluorcyclopenten umsetzt und anschließend die Methylgruppen im erhaltenen 1,2-Bis-(4-methylphenoxy)-3,3,4,4,5,5-hexafluorcyclopenten-1mit Kaliumpermanganat oder Chromtrioxid in $H_2SO_4$ oxidiert.

Eine bevorzugte Ausführungsform des erfindungsgemäßen Verfahrens zur Herstellung von fluorierten Bis-aryloxy-substituierten Alkenen der Formel (I) ist dadurch gekennzeichnet, daß man ein Phenol oder Phenolat der Formel (II), in der ein Rest aus der Gruppe $R_1''$ bis $R_5''$ nicht Wasserstoff bedeutet und einen $\sigma$-Wert von größer als 0,20 aufweist oder in der mehrere Reste aus der Gruppe $R_1''$ bis $R_5''$ nicht Wasserstoff bedeuten und mindestens die Hälfte dieser Reste $\sigma$-Werte von größer als 0,20 aufweisen
mit einem Perhalogenalken der Formel (III) unter Zusatz von 1,7 bis 2,3 Mol einer Base pro Mol der Verbindung der Formel (III) und in Gegenwart eines inerten organischen Lösungsmittels umsetzt und gegebenenfalls anschließend eine Derivatisierung und/oder Umderivatisierung durchführt.

Eine weitere bevorzugte Ausführungsform des erfindungsgemäßen Verfahrens zur Herstellung von fluorierten Bisaryloxy-substituierten Alkenen der Formel (I) ist dadurch gekennzeichnet, daß man ein Phenolat ($R_{11}$ = ein Alkalimetall) der Formel (II), in der ein Rest aus der Gruppe $R_1''$ bis $R_5''$ nicht Wasserstoff bedeutet und einen $\sigma$-Wert von kleiner als 0,20 aufweist oder in der mehrere Reste aus der Gruppe $R_1''$ bis $R_5''$ nicht Wasserstoff bedeuten und mindestens die Hälfte dieser Reste $\sigma$-Werte von kleiner als 0,20 aufweisen mit einem Perhalogenalken der Formel (III) in Gegenwart eines polaren aprotischen Lösungsmittels umsetzt und gegebenenfalls anschließend eine Derivatisierung und/oder Umderivatisierung durchführt.

Die $\sigma$-Werte der verschiedensten Substituenten sind beispielsweise in J.March, Advanced Organic Chemistry, 2nd edition (1977), McGraw-Hill Kogakusha, Seiten 251 ff erläutert und angegeben.

Als aprotische Lösungsmittel kommen z.B. Acetonitril, Dimethylformamid, Tetramethylensulfon, Hexamethylphosphorsäuretriamid, Tetramethylharnstoff oder Propylencarbonat in Frage.

Eine weitere bevorzugte Ausführungsform des erfindungsgemäßen Verfahrens zur Herstellung von fluorierten Bisaryloxy-substituierten Alkenen der Formel (I) ist dadurch gekennzeichnet, daß das Phenol der Formel (II) als Tri-$C_1$-$C_4$-alkylsilylether eingesetzt wird ($R_{11}$ = z.B. Trimethylsilyl), der in situ durch Basen, z.B. Triethylamin, gespalten wird. Dieses Verfahren kann dann vorteilhaft sein, wenn die freien Phenole der Formel (II) instabil oder relativ wenig reaktiv sind.

Schließlich ist auch noch eine besondere Ausführungsform des erfindungsgemäßen Verfahrens zur Herstellung von Bis-aryloxy-substituierten Alkenen der Formel (I) dadurch gekennzeichnet, daß man ein beliebiges Phenol ($R_{11}$ = Wasserstoff) der Formel (II) und ein Perhalogenalken der Formel (III) gemeinsam in einem organischen, mit Wasser nicht mischbaren Lösungsmittel vorlegt und dazu als Base 1,7 bis 2,3 Mol Alkali in Form einer wäßrigen Lösung und einen Phasentransferkatalysator zugibt.

Geeignete Phasentransferkatalysatoren sind z.B. Kronenether wie 1,4,7,10,13,16-Hexacyclooctadecan (18-Krone-6), Dicyclohexyl-18-Krone-6, Dibenzo-18-Krone-6 und ähnliche makrocyclische Polyether (siehe Pedersen, J.A.C.S. 89, 2495 u. 7017 (1967)), sowie quarternäre Ammoniumsalze, z.B. die Tetraalkylammoniumchloride, -bromide, -hydrogensulfate und -hydroxide, die gleiche oder verschiedene Alkylgruppen mit z.B. 1 bis 16 C-Atomen enthalten können und wobei eine Alkylgruppe auch eine Benzylgruppe sein kann.

Die vorliegende Erfindung betrifft weiterhin die Verwendung der neuen fluorierten Bis-aryloxy-substituierten Alkene der Formel (I) als Elektroisoliermittel. Die Verbindungen der Formel (I) sind beispielsweise als Tränkmittel für Kondensatorendielektrika, insbesondere für Hochspannungskondensatoren, und als Flüssigkeit für Transformatorenkühlung verwendbar. Hierfür sind sie aufgrund ihrer Flammfestigkeit (Flammpunkt über 250 °C) und thermischen Stabilität, ihrer niederen Viskosität, der einfachen Zugänglichkeit in hohen Reinheiten, ihres Absorptionsvermögens für bei stillen Entladungen im elektrischen Feld entstehenden Wasserstoff, ihr inertes Verhalten und ihre Dielektrizitätszahlen besonders geeignet.

Beispiele

Beispiel 1

Herstellung von 1,2-Bis-(4-bromphenoxy)-3,3,4,4,5,5-hexafluorcyclopenten-1

86,5 g 4-Bromphenol (0.5 Mol) und 50,5 g Triethylamin wurden in 300 ml Methylenchlorid bei 5°C gelöst. Innerhalb von 25 Minuten wurden bei 5°C 53 g Perfluorcyclopenten (0,25 Mol) zugetropft. Danach wurde die Temperatur auf die Raumtemperatur ansteigen gelassen und 18 Stunden lang bei Raumtemperatur gerührt. Zur Aufarbeitung wurde der Ansatz nacheinander mit 200 ml Wasser, 100 ml 10 gew.-%iger Natronlauge, 100 ml 5 gew.-%iger Salzsäure und nochmals 200 ml Wasser gewaschen, danach die organische Phase getrocknet, eingeengt und fraktioniert destilliert. Bei einem Siedepunkt von 132 bis 135°C bei 0,04 mbar wurden 68 g Produkt erhalten (= 52,5 % der Theorie). Die Massenspektrometrie ergab einen Wert für m/e von 516 für das Molekülion. Das $^{19}$F-Kernresonanzspektrum zeigte charakteristische Banden bei $\delta$ = -36,1 ppm und bei $\delta$ = -51,7 ppm, gemessen gegen Trifluoressigsäure als externen Standard.

Beispiel 2

Herstellung von 1,2-Bis-(3,4-dimethylphenoxy)-3,3,4,4,5,5-hexafluorcyclopenten-1

48,8 g 3,4-Dimethylphenol (0,4 Mol) wurden in 100 ml Methanol gelöst und mit 72 g 30 gew.-%iger Natriummethylatlösung in das entsprechende Natriumsalz überführt. 66 g dieses Natriumsalzes wurden in 150 ml Dimethylformamid gelöst und bei 3 bis 10°C innerhalb von 45 Minuten mit 42,4 g Perfluorcyclopenten (= 0,2 Mol) versetzt. Nach der Erwärmung auf Raumtemperatur wurde 18,5 Stunden bei Raumtemperatur gerührt. Zur Aufarbeitung wurde der Ansatz in 300 ml Wasser eingerührt, die organische Phase abgetrennt, die wäßrige Phase mit 100 ml Methylenchlorid gewaschen, die abgetrennte organische Phase und die Methylenchloridphase vereinigt und nacheinander mit 100 ml Wasser, 50 ml 10 gew.-%iger Natronlauge und nochmals mit 100 ml Wasser gewaschen. Das so erhaltene Rohprodukt wurde aus Toluol umkristallisiert. Es wurden 42 g Produkt (= 50,5 % der Theorie) mit einem Schmelzpunkt von 65 bis 67°C erhalten. Die Massenspektrometrie ergab einen Wert für m/e von 416 für das Molekülion. Das $^{19}$-F-Kernresonanzspektrum zeigte charakteristische Banden bei $\delta$ = -36,0 ppm und bei $\delta$ = -51,8 ppm, gemessen gegen Trifluoressigsäure als externen Standard.

Beispiel 3

Herstellung von 1,2-Bis-[4-(1-methylethen-1-yl)-phenoxy]-3,3,4,4,5,5-hexafluorcyclopenten-1

In 100 ml Methylenchlorid wurden bei 5°C 41,4 g (0,2 Mol) 4-(1-Methylethen-1-yl)-phenol-trimethylsilylether und 20,2 g (0,2 Mol) Triethylamin vorgelegt. Innerhalb von 15 Minuten wurden bei 5°C 21,2 g (0,1 Mol) Perfluorcyclopenten zugetropft und dann die Temperatur auf Raumtemperatur ansteigen gelassen. Anschließend wurde 17 Stunden am Rückfluß (40°C) nachgerührt. Zur Aufarbeitung wurde das Reaktionsgemisch nacheinander mit 100 ml Wasser, 50 ml 5 gew.-%iger Salzsäure und nochmals mit 100 ml Wasser gewaschen. Die Methylenchloridphase wurde über Natriumsulfat getrocknet, filtriert und das Lösungsmittel abgezogen. Das hochviskose Rohprodukt (41 g) wurde im Ölpumpenvakuum destilliert. Es wurden 35 g (= 79,5 % der Theorie) eines hochviskosen Öls mit einem Siedepunkt von 130 bis 145°C bei 0,05 mbar erhalten. Das $^{19}$-F-Kernresonanzspektrum zeigte charakteristische Banden bei $\delta$ = -36,4 ppm und bei $\delta$ = -52,0 ppm, gemessen gegen Trifluoressigsäure als externen Standard.

Beispiel 4

Herstellung von 1,2-Bis-[4-(thiomethyl)phenoxy]-3,3,4,4,5,5-hexafluorcyclopenten-1

70 g 4-Thiomethylphenol (0,5 Mol) wurden in 100 ml Methanol unter Erwärmen gelöst und mit 90 g einer 30 gew.-%igen Natriummethylatlösung versetzt. Das Gemisch wurde am Rotationsverdampfer bis zur Trockene eingedampft, wobei 87 g Natrium-4-thiomethylphenolat hinterblieben. Diese wurden in 150 ml Dimethylformamid gelöst, bei 5°C innerhalb von 45 Minuten mit 53 g Perfluorcyclopenten (= 0,25 Mol) versetzt, dann auf Raumtemperatur erwärmt und 20 Stunden bei Raumtemperatur gerührt. Da nach wurde

der Ansatz auf 300 ml Wasser gegossen, das wäßrige Gemisch filtriert, das Filtrat mit 100 ml Methylenchlorid extrahiert, die Methylenchloridphase abgetrennt, getrocknet, eingeengt und fraktioniert destilliert. Bei einem Siedepunkt von 110 bis 120 °C bei 0,04 mbar wurden 66 g Produkt erhalten (= 58,4 % der Theorie). Das $^{19}F$-NMR-Spektrum zeigte charakteristische Banden bei $\delta$ = -38,8 ppm und bei $\delta$ = -51,5 ppm, gemessen gegen Trifluoressigsäure als externen Standard. Die Massenspektrometrie ergab einen Wert für m/e = 452 für das Molekülion.

Beispiel 5

Herstellung von 1,2-Bis-(4-formylphenoxy)-3,3,4,4,5,5-hexafluorcyclopenten-1

122 g p-Hydroxybenzaldehyd (1 Mol) und 101 g Triethylamin wurden bei 5 °C in 400 ml Methylenchlorid vorgelegt und dazu innerhalb von 30 Minuten 106 g Perfluorcyclopenten (0,5 Mol) zugetropft. Danach wurde auf Raumtemperatur erwärmt und 20,5 Stunden bei Raumtemperatur gerührt. Zur Aufarbeitung wurde der Ansatz in 200 ml Wasser gegeben, die organische Phase abgetrennt und nacheinander mit 100 ml 5 gew.-%iger Salzsäure und 200 ml Wasser gewaschen, getrocknet und am Rotationsverdampfer bis zur Trockene eingedampft. Der Rückstand wurde aus 150 ml Toluol umkristallisiert. So wurden 147 g Produkt mit einem Schmelzpunkt von 98 bis 102 °C erhalten (= 70,7 % der Theorie). Das Massenspektrum ergab einen Wert für m/e = 416 für das Molekülion, das $^{19}F$-NMR-Spektrum charakteristische Banden bei $\delta$ = -36,3 ppm und bei $\delta$ = -51,7 ppm, gemessen gegen Trifluoressigsäure als externen Standard.

Beispiel 6

Herstellung von 2,3-Bis-(4-nitrophenoxy)-1,1,1,4,4,4-hexafluorbuten-2 (cis-trans-Gemisch)

Zu 53,2 g (0,33 Mol) Natrium-p-nitrophenolat, gelöst in 300 ml Dimethylformamid wurden bei 90 °C 0,1 Mol eines Gemisches aus Hexafluordibrombuten-2 und Hexafluorbromchlorbuten-2 zugetropft und 16 Stunden bei dieser Temperatur nachgerührt. Das Reaktionsgemisch wurde mit 500 ml 5 gew.-%iger Natronlauge gewaschen und mit 1,5 l Essigsäureethylester extrahiert. Nach dem Abdestillieren des Essigsäureethylesters wurden 41,5 g Produkt (= 95 % der Theorie) erhalten. Das Massenspektrum ergab einen Wert für m/e von 438 für das Molekülion.

Beispiel 7

Herstellung von 2,3-Bis-(4-aminophenoxy)-1,1,1,4,4,4-hexafluorbuten-2 (cis-trans-Gemisch)

41,0 g (0,094 Mol) der gemäß Beispiel 6 erhaltenen Verbindung wurden in 100 ml Methanol bei 30 bis 40 bar und 40 °C in Gegenwart von 3 g Katalysator (5 Gew.-% Pd auf Kohle) hydriert. Nach dem Abfiltrieren der festen Bestandteile wurde das Lösungsmittel abdestilliert und der Rückstand aus Toluol umkristallisiert. Es wurden 23 g Produkt (= 65 % der Theorie) mit einem Schmelzpunkt von 191 bis 195 °C erhalten. Das $^{19}F$-Kernresonanzspektrum zeigte bei $\delta$ = -14,2 ppm eine charakteristische Bande, gemessen gegen Trifluoressigsäure als externen Standard.

Beispiel 8

Herstellung von 1,2-Bis-(4-nonylphenoxy)-3,3,4,4,5,5-hexafluorcyclopenten-1

121 g Natrium-4-nonylphenolat (0,5 Mol) wurden in 250 ml Dimethylformamid suspendiert und dazu unter Rühren bei 5 bis 10 °C langsam 53 g (0,25 Mol) Perfluorcyclopenten zugegeben (Zugabezeit: 60 Minuten). Anschließend wurde das Gemisch auf Raumtemperatur gebracht und 8 Stunden nachgerührt. Zur Aufarbeitung wurde das Reaktionsgemisch in 400 ml Wasser eingerührt, die sich abscheidende organische Phase abgetrennt und in 200 ml Methylenchlorid aufgenommen. Die Methylenchloridlösung wurde nacheinander mit 100 ml 5 gew.-%iger Natronlauge und 200 ml Wasser gewaschen, dann über Natriumsulfat getrocknet und das Methylenchlorid am Rotationsverdampfer abgezogen. Der verbleibende Rückstand wurde bei 0,05 mbar und 100 °C non niedersiedenden Komponenten befreit. Es wurden 62,5 g Produkt (= 41 % der Theorie) als weißlich trübes, hochviskoses Öl erhalten. Das $^{19}F$-NMR-Spektrum zeigte charakteristische Banden bei $\delta$ = -36,3 ppm und bei $\delta$ = -51,8 ppm, gemessen gegen Trifluoressigsäure als externen Standard.

Beispiel 9

Herstellung von 1,2-Bis-(4-nitrophenoxy)-3,3,4,4-tetrafluorcyclobuten-1

Zu 139 g (1 Mol) 4-Nitrophenol und 101 g (1 Mol) Triethylamin gelöst in 500 ml Methylenchlorid wurden bei 0 bis 5 °C innerhalb von 30 Minuten 81 g (0,5 Mol) Hexafluorcyclobuten eingeleitet. Nach 2 Stunden wurde auf Raumtemperatur erwärmt und weitere 2 Stunden nachgerührt. Zur Aufarbeitung wurde das Reaktionsgemisch nacheinander 2 mal mit je 200 ml Wasser, 1 mal mit 100 ml 5 gew.-%iger Salzsäure und nochmals mit 200 ml Wasser gwaschen, danach über Natriumsulfat getrocknet, das Methylenchlorid am Rotationsverdampfer entfernt und so ein weißer kristalliner Feststoff erhalten. Nach dem Umkristallisieren aus Petrolether wurde das Produkt in einer Ausbeute von 75 % der Theorie mit einem Schmelzpunkt von 112 bis 115 °C erhalten. Das Massenspektrum ergab einen Wert für m/e von 400 für das Molekülion, das $^{19}F$-NMR-Spektrum zeigte eine Bande bei $\delta$ = -35,5 ppm, gemessen gegen Trifluoressigsäure als externen Standard.

Beispiel 10

Herstellung von 1,2-Bis-(4-chlorphenoxy)-3,3,4,4,5,5-hexafluorcyclopenten-1

Zu 128,5 g 4-Chlorphenol (1 Mol) und 101 g Triethylamin (1 Mol) in 300 ml Methylenchlorid wurden bei 0 bis 5 °C 106 g (0,5 Mol) Perfluorcyclopenten innerhalb von 60 Minuten zugetropft. Danach wurde langsam auf Raumtemperatur gebracht und dann 16 Stunden bei 40 °C nachgerührt. Zur Aufarbeitung wurde das Reaktionsgemisch zweimal mit je 200 ml Wasser, danach einmal mit 100 ml 5 %iger Salzsäure und nochmals mit 200 ml Wasser gewaschen, danach über Natriumsulfat getrocknet, Methylenchlorid am Rotationsverdampfer abgezogen und das verbleibende Öl im Vakuum fraktioniert destilliert. Mit einem Siedepunkt von 113 bis 115 °C bei 0,03 mbar wurden 144 g Produkt (= 66 % der Theorie) erhalten. Der Schmelzpunkt des Produkts lag bei 36 bis 37 °C. Das Massenspektrum ergab einen Wert für m/e von 428 ($^{35}Cl$), für das Molekülion. Das $^{19}F$-NMR-Spektrum zeigte Banden bei $\delta$ = -36,1 ppm und bei $\delta$ = -51,7 ppm, gemessen gegen Trifluoressigsäure als externen Standard.

Die Bestimmung der Dielektrizitätszahl bei 100 kHz, 1 V Meßspannung an einer Pastenelektrode bei 40 °C ergab einen Wert von 4,06.

Beispiel 11

Herstellung von 2,3-Bis-[2,5-di(trifluormethyl)-phenoxy]-1,1,1,4,4,4-hexafluorbuten-2 (cis-trans-Gemisch)

Zu 46 g (0,2 Mol) Natrium-2,5-di(trifluormethyl-phenolat, gelöst in 150 ml Dimethylformamid wurden bei 80 °C 28 g (0,1 Mol) Hexafluorbromchlorbuten-2 zugetropft und 4 Stunden bei dieser Temperatur nachgerührt. Das Reaktionsgemisch wurde mit 300 ml 5 gew.-%iger Natronlauge gewaschen und mit 1,5 l Essigsäureethylester extrahiert. Nach dem Abdestillieren des Essigsäureethylesters wurden 54,5 g Produkt (= 88 % der Theorie) erhalten. Das Produkt wurde destillativ gereinigt; der Siedepunkt betrug 63 bis 69 °C bei 0,1 mbar. Das $^{19}$-F-Kernresonanzspektrum zeigte charakteristische Banden bei $\delta$ = +15,4 ppm und +15,0 ppm für die $CF_3$-Gruppen, gemessen gegen Trifluoressigsäure als externen Standard. Im $^{1}$-H-Kernresonanzspektrom lagen die Protonenresonanzen des Aromaten bei $\delta$ = 7,5 ppm und $\delta$ = 7,75 ppm, gemessen gegen Tetramethylsilan als internen Standard.

Beispiel 12

Herstellung von 2,3-Bis-[4-(methyloxycarbonyl)-phenoxy]-1,1,1,4,4,4-hexafluorbuten-2 (cis-trans-Gemisch)

Zu 30,4 g (0,2 Mol) Natrium-4-(methyloxycarbonyl)-phenolat, gelöst in 150 ml Dimethylformamid wurden bei 80 °C 28 g (0,1 Mol) Hexafluorbromchlorbuten-2 zugetropft und 4 Stunden bei dieser Temperatur nachgerührt. Das Reaktionsgemisch wurde mit 300 ml 5 gew.-%iger Natronlauge gewaschen, mit 1,5 l Essigsäureethylester extrahiert, der Essigsäureethylester abdestilliert und danach 44 g Produkt (= 95 % der Theorie) mit einem Schmelzpunkt von 123 °C erhalten. Das Massenspektrum ergab einen Wert für m/e von 464 für das Molekülion.

Beispiel 13

Herstellung von 1,2-Bis-(4-fluorphenoxy)-3,3,4,4,5,5-hexafluorcyclopenten-1

112 g 4-Fluorphenol (1 Mol) und 101 g Triethylamin wurden in 350 ml Methylenchlorid bei +5°C gelöst. Innerhalb von 45 Minuten wurden bei 5°C 106 g Perfluorcyclopenten (0,5 Mol) zugetropft. Danach wurde die Temperatur auf Raumtemperatur ansteigen gelassen und 18 Stunden lang bei Raumtemperatur gerührt. Zur Aufarbeitung wurde der Ansatz nacheinander mit 200 ml Wasser, 100 ml 5 gew.-%iger Salzsäure und nochmals 200 ml Wasser gewaschen, danach die organische Phase getrocknet, eingeengt und fraktioniert destilliert. Bei einem Siedepunkt von 98 bis 108°C bei 0,04 mbar wurden 125 g Produkt erhalten (= 63 % der Theorie), das in der Vorlage zu einem Feststoff mit einem Schmelzpunkt von 79 bis 83°C erstarrte. Das Massenspektrum ergab einen Wert für m/e von 396 für das Molekülion.

Beispiel 14

Herstellung von 1,2-Bis-(2,4-dichlorphenoxy)-3,3,4,4,5,5-hexafluorcyclopenten-1

Zu 326 g 2,4-Dichlorphenol (2 Mol) und 202 g Triethylamin (2 Mol) in 500 ml Methylenchlorid wurden bei 0 bis 5°C 212 g (1 Mol) Perfluorcyclopenten innerhalb von 90 Minuten zugetropft. Danach wurde langsam auf Raumtemperatur gebracht und dann 16 Stunden bei 40°C nachgerührt. Zur Aufarbeitung wurde das Reaktionsgemisch zweimal mit je 200 ml Wasser, danach mit 200 ml 5 gew.-%iger Salzsäure und nochmals mit 200 ml Wasser gewaschen, danach über Natriumsulfat getrocknet, Methylenchlorid am Rotationsverdampfer abgezogen und das verbleibende Öl im Vakuum fraktioniert destilliert. Mit einem Siedepunkt von 136 bis 138°C bei 0,03 mbar wurden 424 g Produkt (= 85 % der Theorie) erhalten. Das Massenspektrum ergab einen Wert für m/e von 496 ($^{35}Cl$), für das Molekülion. Das $^{19}F$-Kernresonanzspektrum zeigte charakteristische Banden bei $\delta$ = -35,6 ppm und bei $\delta$ = -51,7 ppm, gemessen gegen Trifluoressigsäure als externen Standard.

Beispiel 15

Herstellung von 1,2-Bis-(2,4,5-trichlorphenoxy)-3,3,4,4,5,5-hexafluorcyclopenten-1

Zu 790 g 2,4,5-Trichlorphenol (4 Mol) und 404 g Triethylamin (4 Mol) in 1800 ml Methylenchlorid wurden bei 0 bis 5°C 424 g (2 Mol) Perfluorcyclopenten innerhalb von 120 Minuten zugetropft. Danach wurde langsam auf Raumtemperatur gebracht und dann 16 Stunden bei 40°C nachgerührt. Zur Aufarbeitung wurde das Reaktionsgemisch zweimal mit je 500 ml Wasser, danach einmal mit 500 ml 5 gew.-%iger Salzsäure und nochmals mit 500 ml Wasser gewaschen, danach über Natriumsulfat getrocknet, Methylenchlorid am Rotationsverdampfer abgezogen und der verbleibende ölige Rückstand aus wenig Methylenchlorid umkristallisiert. Es wurden 845 g Produkt (= 75 % der Theorie) mit einem Schmelzpunkt von 65 bis 68°C erhalten. Das Massenspektrum ergab einen Wert für m/e von 564 ($^{35}Cl$), für das Molekülion. Das $^{19}F$-Kernresonanzspektrum zeigte Banden bei $\delta$ = -35,5 ppm und bei $\delta$ = -51,5 ppm, gemessen gegen Trifluoressigsäure als externen Standard.

Beispiel 16

Herstellung von 1,2-Bis-[4-(methyloxycarbonyl)phenoxy]-3,3,4,4,5,5-hexafluorcyclopenten-1

Zu 456 g p-Hydroxybenzoesäuremethylester (3 Mol) und 303 g Triethylamin (3 Mol) in 1000 ml Methylenchlorid wurden bei 5 bis 10°C 320 g Perfluorcyclopenten (1,51 Mol) aus einem gekühlten Tropftrichter unter Rühren innerhalb von 60 Minuten zugetropft. Nach der Erwärmung auf Raumtemperatur wurde noch 16 Stunden bei 40°C nachgerührt, dann das Reaktionsgemisch zweimal mit je 300 ml Wasser, einmal mit 200 ml 5 gew.-%iger Salzsäure und nochmals mit 300 ml Wasser gewaschen. Danach wurde das Gemisch über Natriumsulfat getrocknet, das Methylenchlorid am Rotationsverdampfer abgezogen und so 675 g Rohprodukt (95 % der Theorie) mit einem Schmelzpunkt von 67 bis 74°C erhalten. Das Rohprodukt wurde aus 250 ml Toluol umkristallisiert, wobei sich eine erste Kristallfraktion von 360 g Produkt mit einem Schmelzpunkt von 88 bis 92°C und eine zweite Kristallfraktion von 236,5 g mit einem Schmelzpunkt von 85 bis 91°C ergab. Das entspricht einer Gesamtausbeute an umkristallisiertem Produkt von 83 %. Das Massenspektrum für dieses Produkt ergab einen Wert für m/e von 476 für das Molekülion.

Beispiel 17

Herstellung von 1,2-Bis-(4-methylphenoxy)-3,3,4,4,5,5-hexafluorcyclopenten-1

Zu 150,5 g (1 Mol) Natrium-4-methylphenolat, gelöst in 300 ml Dimethylformamid, wurden bei 0 bis 5 ° C langsam 106 g Perfluorcyclopenten (0,5 Mol) innerhalb von 60 Minuten zugetropft. Nach Abklingen der leicht exothermen Reaktion wurde innerhalb von 2 Stunden auf Raumtemperatur erwärmt und dann 4 Stunden bei Raumtemperatur nachgerührt. Das Reaktionsgemisch wurde in 500 ml Wasser eingerührt, die organische Phase mit 250 ml Methylenchlorid aufgenommen, die Methylenchloridlösung mit 100 ml 5 gew.-%iger Natronlauge und anschließend mit 200 ml Wasser gewaschen, über Natriumsulfat getrocknet und am Rotationsverdampfer eingeengt. Das zurückbleibende Öl wurde fraktioniert destilliert. Mit einem Siedepunkt von 104 bis 108 ° C bei 0,2 mbar und mit einem Schmelzpunkt von 46 bis 48 ° C wurden 130 g Produkt erhalten (= 67 % der Theorie). Das Massenspektrum ergab einen Wert für m/e von 388 für das Molekülion. Das $^{19}$F-NMR-Spektrum zeigte Banden bei $\delta$ = -35,9 ppm und bei $\delta$ = -51,8 ppm, gemessen gegen Trifluoressigsäure als externen Standard.

Beispiel 18

Herstellung von 1,2-Bis-[2-(trifluormethyl)-4-nitrophenoxy]-3,3,4,4,5,5-hexafluorcyclopenten-1

Zu 138 g (0,66 Mol) 2-Trifluormethyl-4-nitrophenol und 66,7 g Triethylamin (0,66 Mol) in 300 ml Methylenchlorid wurden bei 0 bis 5 ° C unter Rühren innerhalb von 30 Minuten 70 g (0,33 Mol) Perfluorcyclopenten aus einem gekühlten Tropftrichter zugetropft. Nach 30 Minuten wurde langsam auf Raumtemperatur erwärmt und 3 Stunden bei Raumtemperatur nachgerührt. Das Reaktionsgemisch wurde nacheinander zweimal mit je 200 ml Wasser, einmal mit 100 ml 5 %iger Salzsäure und abschließend nochmals mit 100 ml Wasser gewaschen und über Natriumsulfat getrocknet. Nach dem Abziehen des Lösungsmittels verblieb ein weißlich-braunes Rohprodukt in einer Menge von 135 g (= 70 % der Theorie) mit einem Schmelzpunkt von 95 bis 110 ° C. Die Umkristallisation aus Petrolether lieferte 85 % dieses Produkts zurück mit einem Schmelzpunkt von 118 bis 120 ° C. Das Massenspektrum ergab einen Wert für m/e von 586 für das Molekülion. Das $^{19}$F-NMR-Spektrum zeigte Banden bei $\delta$ = +16,1 ppm ($CF_3$), -35,2 ppm und -51,7 ppm, gemessen gegen Trifluoressigsäure als externen Standard.

Beispiel 19

Herstellung von 1,2-Bis-[2-(trifluormethyl)-4-aminophenoxy]-3,3,4,4,5,5-hexafluorcyclopenten-1

393 g (0,5 Mol) eines gemäß Beispiel 18 hergestellten Produktes wurden in 800 ml Dioxan bei 35 bis 40 ° C und 30 bis 40 bar Wasserstoff in einem 1,7 l-Edelstahlautoklaven 6 Stunden in Gegenwart von 20 g Raney-Nickel hydriert. Danach wurde das Hydriergemisch filtriert, aus dem Filtrat das Dioxan im Vakuum entfernt und so 260 g rohes Produkt (= 98,8 % der Theorie) mit einem Schmelzpunkt von 88 bis 93 ° C erhalten. 100 g dieses Produktes wurden aus 200 ml Petrolether umkristallisiert. Der Rückgewinn betrug 85 %, der Schmelzpunkt betrug dann 102 bis 104 ° C. Das Massenspektrum des gereinigten Produktes ergab einen Wert für m/e von 526 für das Molekülion.

Beispiel 20

Darstellung von 1,2-Bis-(4-carboxylphenoxy)-3,3,4,4,5,5-hexafluorcyclopenten-1

135 g (0,28 Mol) des Produkts aus Beispiel 16 wurden in einem Gemisch aus 450 ml Dioxan und 100 ml Wasser gelöst und nach Zugabe von 25 g gepulvertem Natriumhydroxid (0,625 Mol) 30 Minuten bei 80 bis 90 ° C gerührt. Anschließend wurde auf Raumtemperatur abgekühlt und langsam 50 ml konzentrierte Salzsäure zugesetzt. Das ausfallende Produkt wurde abgesaugt, mit leicht angesäuertem Wasser gewaschen und bei 110 ° C 2 Stunden getrocknet. Die Rohausbeute betrug 118 g (= 94 % der Theorie) und wies einen Schmelzpunkt von 243 bis 246 ° C auf. Nach dem Umkristallisieren aus einem aus etwa gleichen Volumenteilen Ethanol und Wasser bestehenden Gemisch betrug die Ausbeute an Produkt 102 g (= 80 % der Theorie) und der Schmelzpunkt 246 bis 248 ° C. Das Massenspektrum des gereinigten Produktes ergab einen Wert von m/e von 448 für das Molekülion. Das $^{19}$F-NMR-Spektrum zeigte Banden bei $\delta$ = -34,9 ppm und -50,7 ppm, gemessen gegen Trifluoressigsäure als externem Standard.

Beispiel 21

Herstellung von 1,2-Bis-(4-nitrophenoxy)-3,3,4,4,5,5-hexafluorcyclopenten-1

a) In einem 2 l-Dreihalskolben wurden 278 g (2 Mol) 4-Nitrophenol und 202 g Triethylamin (2 Mol) in 800 ml Methylenchlorid bei 5 bis 10 °C vorgelegt. Dazu wurden aus einem gekühlten Tropftrichter unter Rühren innerhalb von 60 Minuten 212 g Perfluorcyclopenten (1 Mol) zugetropft. Nach etwa 70 % der Zugabe begann die Niederschlag auszufallen, der sich nach dem Erwärmen auf 25 °C wieder auflöste. Es wurde 4 Stunden bei Raumtemperatur nachgerührt, dann wurde die Methylenchloridlösung nacheinander zweimal mit je 300 ml Wasser, einmal mit 200 ml 5 %iger Salzsäure und nochmals mit 300 ml Wasser gewaschen, über Natriumsulfat getrocknet und das Methylenchlorid am Rotationsverdampfer bis zur Trockene abgezogen. Es hinterblieben 425 g Rohprodukt (= 94,4 % der Theorie) mit einem Schmelzpunkt von 117 bis 121 °C. Nach der Kristallisation aus Methylenchlorid betrug der Schmelzpunkt des Produktes 124 bis 127 °C. Das Massenspektrum ergab einen Wert für m/e von 450 für das Molekülion. Das $^{19}$F-NMR-Spektrum zeigte Banden bei $\delta$ = -35,8 ppm und -51,4 ppm, gemessen gegen Trifluoressigsäure als externem Standard.

b) In 50 ml Dimethylformamid wurden 24,5 g 1,2-Dichlor-3,3,4,4,5,5-hexafluorcyclopenten-1 (0,1 Mol) bei 10 °C vorgelegt. Nach dem Zusatz von 27,8 g 4-Nitrophenol (0,2 Mol) und 11,2 g (0,2 Mol) fein gepulvertem KOH wurde der Ansatz unter Rühren langsam auf 50 °C erwärmt und 20 Stunden bei dieser Temperatur gehalten. Danach wurde das Reaktionsgemisch in 500 ml Wasser eingegossen, Methylenchlorid hinzugefügt, die organische Phase abgetrennt und nacheinander mit 100 ml Wasser Wasser, 100 ml 10 Gew.-%iger Natronlauge und nochmals mit 100 ml Wasser gewaschen. Nach dem Trocknen über Natriumsulfat wurde am Rotationsverdampfer bis zur Trockene eingeengt. Rohausbeute: 26 g, das entspricht 57,8 % der Theorie.

Nach der Umkristallisation aus Petrolether wurden 22 g gereinigtes Produktes mit einem Schmelzpunkt von 118 bis 123 °C erhalten, dessen spektroskopische Daten (Massenspektrum und $^{19}$F-NMR-Spektrum) mit denen des Produktes aus Beispiel 15a) identisch waren.

Beispiel 22

Herstellung von 1,2-Bis-(4-aminophenoxy)-3,3,4,4,5,5-hexafluorcyclopenten-1

420 g des gereinigten Produktes aus Beispiel 21 wurden in 1600 ml Dioxan bei 35 bis 40 °C und 30 bis 40 bar Wasserstoff in einem 3 l-Edelstahlautoklaven 5 Stunden in Gegenwart von 40 g Raney-Nickel hydriert. Danach wurde das Hydriergemisch filtriert, aus dem Filtrat das Dioxan im Vakuum entfernt und so 358 g rohes Produkt (= 91,8 % der Theorie) mit einem Schmelzpunkt von 78 bis 83 °C erhalten. 50 g dieses Produktes wurden aus 200 ml Cyclohexan kristallisiert. Der Rückgewinn betrug 75 %, der Schmelzpunkt betrug dann 94 bis 97 °C. Das Massenspektrum des gereinigten Produktes ergab einen Wert für m/e von 390 für das Molekülion. Das $^{19}$F-NMR-Spektrum zeigte Banden bei $\delta$ = -35,5 ppm und -51,7 ppm, gemessen gegen Trifluoressigsäure als externem Standard.

Beispiel 23

Herstellung von 1,2-Bis-(4-isocyanatophenoxy)-3,3,4,4,5,5-hexafluorcyclopenten-1

195 g (0,5 Mol) von gemäß Beispiel 22 hergestelltem und gereinigtem Produkt wurden in 100 ml Chlorbenzol gelöst, und bei 0 °C zu einer gut gerührten Lösung von 220 g (2,22 Mol) Phosgen in 1000 ml Chlorbenzol innerhalb von 75 Minuten zugetropft. Nach Beendigung der Kaltphosgenierung wurde innerhalb von 30 Minuten auf 70 °C erhitzt und weiter Phosgen eingeleitet. Danach wurde die Temperatur bis auf 120 °C erhöht und nach 1 Stunde Kochen am Rückfluß die Heißphosgenierung abgeschlossen. Ab 50 °C lag eine klare Lösung vor, ab 80 bis 90 °C setzte starke Chlorwasserstoffgasentwicklung ein. Nach Beendigung der Phosgenierung wurde überschüssiges Phosgen durch Ausblasen mit Stickstoff vertrieben und das Chlorbenzol im Wasserstrahlvakuum abdestilliert. Die Ausbeute an Rohprodukt betrug 212 g (= 96 % der Theorie). Der Schmelzpunkt das Rohprodukts betrug 66 bis 71 °C; es wies einen Gehalt an aktivem NCO von 87 % auf.

Die Reinigung des Produktes durch fraktionierte Destillation ergab mit 75 % Rückgewinn ein Produkt mit einem Siedepunkt von 153 bis 154 °C bei 0,01 mbar, einem Schmelzpunkt von 77 bis 79 °C, einem Gehalt an aktivem NCO von 98,4 % und wies ein Massenspektrum mit einem Wert für m/e von 442 für das

Molekülion auf.

Beispiel 24

Herstellung von 1,2-Bis-[4-(trans-2-carboxyethyl)-ethenylphenoxy]-3,3,4,4,5,5-hexafluorcyclopenten-1

In 20 ml Dimethylformamid wurden unter Stickstoff 5,2 g (0,01 Mol) 1,2-Bis-(4-bromphenoxy)-3,3,4,4,5,5-hexafluorcyclopenten-1 (hergestellt nach Beispiel 1), 2,5 g (0,025 Mol) Acrylsäureethylester, 2,05 g wasserfreies Natriumacetat, 22,5 mg Palladium(II)-acetat und 122,5 mg Tri-o-tolyl-phosphin gegeben. Nach 6 Stunden Kochen am Rückfluß unter Stickstoffatmosphäre wurde das Reaktionsgemisch heiß filtriert. Aus dem Filtrat wurde das Lösungsmittel im Vakuum abgezogen und der Rückstand in Methylenchlorid aufgenommen. Nach zweimaligem Ausschütteln der Methylenchlorid-Phase mit Wasser wurde sie über Natriumsulfat getrocknet. Nach dem Abziehen des Methylenchlorids und dem Umkristallisieren des Rückstandes aus n-Hexan/Ethanol wurden 1,5 g Produkt, das entspricht 27 % der Theorie, mit einem Schmelzpunkt von 117 bis 119 °C erhalten.

Das $^1$H-NMR-Spektrum zeigte charakteristische Banden bei 1,33 ppm, 4,25 ppm, 6,29 ppm, 7,65 ppm, 7,28 ppm und 7,53 ppm, gemessen gegen Tetramethylsilan als internen Standard.

Beispiel 25

Herstellung von 1,2-Bis-[4-(2-hydroxy-2-methylbutin-4-yl)phenoxy]-3,3,4,4,5,5-hexafluorcyclopenten-1

Zu 100 ml Triethylamin wurden unter Stickstoff 39 g (0,075 Mol) 1,2-Bis-(4-bromphenoxy)-3,3,4,4,5,5-hexafluorcyclopenten-1 (erhalten gemäß Beispiel 1), 32 g (0,38 Mol) 2-Methyl-3-butin-2-ol, 211 mg $PdCl_2(PPh_3)_2$, 230 mg CuJ and 630 mg Triphenylphosphin gegeben. Nach 24 Stunden Kochen am Rückfluß wurde das Reaktionsgemisch filtriert und das Filtrat im Vakuum eingeengt. Die Umkristallisation des dabei anfallenden Rückstandes aus n-Hexan/Essigsäureethylester ergab 19,1 g Produkt, das entspricht 48 % der Theorie, mit einem Schmelzpunkt von 108 bis 113 °C. Das $^1$H-NMR-Spektrum zeigte charakteristische Banden bei 1,59 ppm, 6,63 ppm und 7,2 ppm, gemessen gegen Tetramethylsilan als internen Standard.

Beispiel 26

Herstellung von 1,2-Bis-(4-ethinylphenoxy)-3,3,4,4,5,5-hexafluorcyclopenten-1

10,5 g (0,02 Mol) des Produktes aus Beispiel 25 wurden zusammen mit 0,6 g Natriumhydroxid in 50 ml Toluol 2 Stunden erhitzt, wobei ein Gemisch aus Toluol und Aceton abdestillierte. Anschließend wurde das verbleibende Reaktionsgemisch filtriert und das Filtrat im Vakuum eingeengt. Es wurden 8 g Produkt erhalten, dessen $^1$H-NMR-Spektrum charakteristische Banden bei 3,05 ppm, 6,63 ppm und 7,28 ppm aufwies. Die Ausbeute betrug 98 % der Theorie.

Beispiel 27

Herstellung von 1,2-Bis-(4-cyanophenoxy)-3,3,4,4,5,5-hexafluorcyclopenten-1

In 200 ml Methylenchlorid wurden 71,4 g (0,6 Mol) p-Hydroxybenzonitril und 60,6 g Triethylamin bei 5 °C vorgelegt. Innerhalb von 30 Minuten wurden 63,6 g (0,3 Mol) Perfluorcyclopenten zugetropft und anschließend 19,5 Stunden bei Raumtemperatur gerührt. Der Ansatz wurde nacheinander mit 200 ml Wasser, 100 ml 5 gew.-%iger Salzsäure und nochmals mit 100 ml Wasser gewaschen, getrocknet und zur Trockene eingeengt. Die Rohausbeute an Produkt betrug 118,5 g. Die Umkristallisation aus 100 ml Toluol lieferte 107 g gereinigtes Produkt mit einem Schmelzpunkt von 118 bis 121 °C. Die Massenspektrometrie ergab einen Wert für m/e von 410 für das Molekülion. Das $^{19}$F-Kernresonanzspektrum zeigte charakteristische Banden bei $\delta$ = -35,9 ppm und bei $\delta$ = -51,7 ppm, gemessen gegen Trifluoressigsäure als externen Standard.

Beispiel 28

Herstellung von 1,2-Bis[4-(acetamino)phenoxy]-3,3,4,4,5,5-hexafluorcyclopenten-1

Zu 151 g (1 Mol) p-Acetaminophenol und 101 g Triethylamin in 300 ml Methylenchlorid wurden bei 5 °C innerhalb von 30 Minuten 106 g (0,5 Mol) Perfluorcyclopenten zugetropft. Es wurde 3 Stunden bei Raumtemperatur und anschließend noch 17 Stunden bei 40 °C nachgerührt. Danach wurde der ausgefallene Feststoff aus dem Reaktionsgemisch filtriert, der abgetrennte Feststoff nacheinander mit 250 ml Wasser, 250 ml 5 gew.-%iger Salzsäure und nochmals mit 250 ml Wasser gewaschen und bei Raumtemperatur getrocknet. Die Rohausbeute betrug 217 g Produkt, das entspricht 91,6 % der Theorie, und wies einen Schmelzpunt von 198 bis 225 °C auf.

Das Rohprodukt wurde 3 Stunden lang in 300 ml siedendem Methylenchlorid digeriert, erneut abfiltriert und bei 100 °C getrocknet. Es wurden 193 g gereinigtes Produkt mit einem Schmelzpunkt von 230 bis 234 °C erhalten. Das gereinigte Produkt wurde massenspektroskopisch untersucht, wobei sich ein Wert für m/e von 474 für das Molekülion ergab.

Beispiel 29

Herstellung von 1,2-Bis-[4-(carbonylbenzyl)-phenoxy]-3,3,4,4,5,5-hexafluorcyclopenten-1

21 g 4-Hydroxyphenylbenzylketon (0,1 Mol) und 10,1 g Triethylamin wurden bei 5 °C in 50 ml Methylenchlorid vorgelegt und 10,5 g (0,05 Mol) Perfluorcyclopenten zugetropft. Danach wurde auf Raumtemperatur erwärmt und 17 Stunden bei Raumtemperatur gerührt. Zur Aufarbeitung wurde der Ansatz in 50 ml Wasser gegeben, die organische Phase abgetrennt und nacheinander mit 50 ml 5 gew.-%iger Salzsäure und 50 ml Wasser gewaschen, getrocknet und am Rotationsverdampfer bis zur Trockene eingedampft. Der Rückstand wurde aus 50 ml Toluol umkristallisiert. So wurden 18 g Produkt (= 60,4 % der Theorie) mit einem Schmelzpunkt von 97 bis 104 °C erhalten. Das Massenspektrum ergab einen Wert für m/e von 596 für das Molekülion. Das $^{19}F$-Kernresonanzspektrum zeigte Banden bei $\delta$ = -36,3 ppm und bei $\delta$ = 51,6 ppm, gemessen gegen Trifluoressigsäure als externen Standard.

Beispiel 30

Herstellung von 1,2-Bis-[2,5-di(trifluormethyl)phenoxy]-3,3,4,4,5,5-hexafluorcyclopenten-1

Zu 138 g 2,5-Di(trifluormethyl)phenol (0,6 Mol) und 60,6 g Triethylamin in 300 ml Methylenchlorid wurden bei 0 bis 5 °C 63,6 g (0,3 Mol) Perfluorcyclopenten innerhalb von 30 Min. zugetropft. Danach wurde langsam auf Raumtemperatur gebracht und anschließend 16 Stunden bei 40 °C nachgerührt. Zur Aufarbeitung wurde das Reaktionsgemisch mit 400 ml Wasser versetzt, worauf das Produkt ausfiel und abgesaugt wurde. Nach dem Aufnehmen in Toluol wurde mit Wasser gewaschen und über Natriumsulfat getrocknet. Anschließend wurde das Toluol am Rotationsverdampfer abgezogen und der verbleibende Rückstand im Vakuum fraktioniert destilliert. Mit einem Siedepunkt von 80 bis 85 °C bei 0,03 mbar wurden 132 g Produkt (= 69,6 % der Theorie) erhalten. Der Schmelzpunkt des Produktes lag bei 76 bis 79 °C. Das Massenspektrum ergab einen Wert für m/e von 632 für das Molekülion. Das $^{19}F$-NMR-Spektrum zeigte banden bei $\delta$ = -36,7 ppm, bei $\delta$ = -52,5 ppm und bei $\delta$ = +14,1 ppm ($CF_3$), gemessen gegen Trifluoressigsäure als externen Standard.

Die Bestimmung der Dielektrizitätszahl bei 100 kHz, 1 V-Meßspannung an einer Pastenelektrode bei 80 °C ergab einen Wert von 2,20.

Beispiel 31

Herstellung von 1,2-Bis-(4-methoxyphenoxy)-3,3,4,4,5,5-hexafluorcyclopenten-1

49,6 g 4-Hydroxyanisol (0,4 Mol) wurden in 50 ml Methanol unter Erwärmen gelöst und mit 72 g einer 30 gew.-%igen Natriummethylatlösung versetzt. Das Gemisch wurde am Rotationsverdampfer bis zur Trockene eingedampft und das erhaltene Natriumsalz in 150 ml Dimethylformamid gelöst. Bei 5 °C wurden innerhalb von 45 Minuten 42,4 g Perfluorcyclopenten (0,2 Mol) zugetropft, dann wurde auf Raumtemperatur erwärmt und 20 Stunden bei 40 °C nachgerührt. Danach wurde der Ansatz auf 300 ml Wasser gegossen und das wäßrige Gemisch filtriert. Es verblieben 40 g eines pulvrigen Filterkuchens, der in Ethanol teilweise

unlöslich war. Die ethanolische Lösung wurde zur Trockene eingedampft und so 18 g eines teilkristallinen Öls erhalten. Das wäßrige Filtrat wurde mit 100 ml Methylenchlorid extrahiert, die Methylenchloridphase abgetrennt, über Natriumsulfat getrocknet und am Rotationsverdampfer zur Trockene eingeengt. Es wurden weitere 11 g eines Öls erhalten. Beide Produktfraktionen wurden vereinigt und fraktioniert destilliert. Bei einem Siedepunkt von 135 bis 145 °C bei 0,03 mbar wurden 21 g Produkt erhalten (= 25 % der Theorie). Das $^{19}F$-NMR-Spektrum zeigte charakteristische Banden bei $\delta$ = -35,6 ppm und bei $\delta$ = -51,6 ppm, gemessen gegen Trifluoressigsäure als externen Standard. Die Massenspektrometrie ergab einen Wert für m/e von 420 für das Molekülion.

Beispiel 32

Herstellung von 1,2-Bis-(3-nitrophenoxy)-3,3,4,4,5,5-hexafluorcyclopenten-1

In einem 1 l-Dreihalskolben wurden 139 g (1 Mol) 3-Nitrophenol und 101 g Triethylamin (1 Mol) in 400 ml Methylenchlorid bei 5 bis 10 °C vorgelegt. Dazu wurden aus einem gekühlten Tropftrichter unter Rühren innerhalb von 60 Minuten 106 g (0,5 Mol) Perfluorcyclopenten zugetropft. Es wurde 16 Stunden bei Raumtemperatur nachgerührt, dann wurde die Methylenchloridlösung nacheinander zweimal mit je 200 ml Wasser, einmal mit 100 ml 5 %iger Salzsäure und nochmals mit 200 ml Wasser gewaschen, über Natriumsulfat getrocknet und das Methylenchlorid am Rotationsverdampfer bis zur Trockene abgezogen. Es hinterblieben 208 g Rohprodukt (= 92,4 % der Theorie) mit einem Schmelzpunkt von 73 bis 77 °C. Nach der Umkristallisation aus Ligroin wurden 90 % des Produkts wiedergewonnen, der Schmelzpunkt betrug dann 79 bis 83 °C. Das $^{19}F$-NMR-Spektrum zeigte charakteristische Banden bei $\delta$ = -35,7 ppm und bei $\delta$ = -51,5 ppm, gemessen gegen Trifluoressigsäure als externen Standard.

Beispiel 33

Herstellung von 1,2-Bis-(3-aminophenoxy)-3,3,4,4,5,5-hexafluorcyclopenten-1

325 g (0,5 Mol) eines gemäß Beispiels 32 erhaltenen Rohproduktes wurden in 800 ml Dioxan bei 35 bis 40 °C und 30 bis 40 bar Wasserstoff in einem 3 l-Edelstahlautoklaven in Gegenwart von 20 g Raney-Nickel hydriert. Danach wurde das Hydriergemisch filtriert, aus dem Filtrat das Dioxan im Vakuum entfernt und so 185 g rohes Produkt (= 94,9 % der Theorie) als hochviskoses Öl erhalten. 150 g dieses Produktes wurden aus 300 ml Ethanol durch Zugabe von 100 ml Wasser umgefällt. Es wurden 135 g eines hellgelben Öles zurückgewonnen, was 90 % entspricht. Das Massenspektrum des umgefällten Produktes ergab einen Wert für m/e von 390 für das Molekülion.

Beispiel 34

Herstellung von 1-(4-Cyanophenoxy)-2-[4-(methoxycarbonyl)phenoxy]-3,3,4,4,5,5-hexafluorcyclopenten-1

15,2 g (0,1 Mol) 4-Hydroxybenzoesäuremethylester und 10,1 g Triethylamin in 100 ml Methylenchlorid wurden bei 5 °C 21,2 g Perfluorcyclopenten in 15 Min. zugetropft. Nach 4 Stunden Rühren bei Raumtemperatur wurde eine Lösung von 11,9 g (0,1 Mol) 4-Hydroxybenzonitril und 10,1 g Triethylamin in 100 ml Methylenchlorid zum Reaktionsgemisch getropft und weitere 16 Stunden bei 40 °C gerührt. Die Methylenchloridlösung wurde anschließend nacheinander mit 100 ml Wasser, 50 ml 5 %iger Salzsäure und nochmals 100 ml Wasser gewaschen, über Natriumsulfat getrocknet und am Rotationsverdampfer zur Trockene eingedampft. Die Rohausbeute betrug 40,6 g. Das Produkt wurde aus Toluol umkristallisiert und so 39,0 g kristallines Material mit einem Schmelzpunkt von 96 bis 98 °C erhalten, das entspricht 88 % der Theorie.

Das Massenspektrum zeigte einen Wert für m/e von 443 für das Molekülion, das $^{19}F$-NMR-Spektrum zeigte charakteristische Banden bei $\delta$ = -36,1 ppm, $\delta$ = -36,6 ppm und $\delta$ = -51,7 ppm, gemessen gegen Trifluoressigsäure als externen Standard.

Beispiel 35

Herstellung von 1-(4-Bromphenoxy)-2-[4-(methyloxycarbonyl)phenoxy]-3,3,4,4,5,5-hexafluorcyclopenten-1

Zu 17,3 g (0,1 Mol) 4-Bromphenol und 10,1 g Triethylamin in 100 ml Methylenchlorid wurden bei 5 ° C 21,2 g (0,1 Mol) Perfluorcyclopenten innerhalb von 15 Min. zugetropft und 4 Stunden bei Raumtemperatur nachgerührt. Dann wurde eine Lösung von 0,1 Mol 4-Hydroxybenzoesäuremethylester und 10,1 g Triethylamin in 100 ml Methylenchlorid zum Reaktionsgemisch getropft und weitere 16 Stunden bei 40 ° C nachgerührt. Die Methylenchloridlösung wurde anschließend mit 100 ml Wasser, danach 50 ml 5 %iger Salzsäure und nochmals 100 ml Wasser gewaschen, über Natriumsulfat getrocknet und am Rotationsverdampfer zur Trockene eingedampft. Es wurden 48 g eines Öls erhalten, das im Vakuum destilliert wurde. Dabei wurden mit einem Siedepunkt von 128 bis 140 ° C bei 0,02 mbar 41 g Produkt erhalten, was 82,7 % der Theorie entspricht.

Das Massenspektrum zeigte einen Wert für m/e von 496 ($^{79}$Br) als Molekülion. Im $^{19}$F-NMR-Spektrum traten charakteristische Banden bei $\delta$ = -34,4 ppm, $\delta$ = -35,0 ppm und $\delta$ = -50,6 ppm auf, gemessen gegen Trifluoressigsäure als externen Standard.

Beispiel 36

Herstellung von 1-(4-Methylphenoxy)-2-(4-nitrophenoxy)-3,3,4,4,5,5-hexafluorcyclopenten-1

Zu 13 g (0,1 Mol) Natrium-4-methylphenolat in 50 ml Dimethylformamid wurden bei 5 ° C 21,2 g (0,1 Mol) Perfluorcyclopenten innerhalb von 15 Minuten zugetropft. Nach 8 Stunden Rühren bei Raumtemperatur wurde eine Lösung von 13,9 g (0,1 Mol) 4-Nitrophenol und 10,1 g Triethylamin in 50 ml Dimethylformamid zum Reaktionsgemisch getropft und weitere 6 Stunden bei 40 ° C gerührt. Danach wurde das Reaktionsgemisch in 300 ml Wasser eingerührt und die organische Phase nach Zusatz von 100 ml Methylenchlorid abgetrennt. Anschließend wurde die organische Phase nacheinander mit 50 ml Wasser, 50 ml %iger Salzsäure und nochmals 50 ml Wasser gewaschen, über Natriumsulfat getrocknet und am Rotationsverdampfer zur Trockene eingedampft. Die Rohausbeute an Produkt betrug 34 g. Es wurde aus Petrolether umkristallisiert und so 20 g kristallines Material erhalten, was 47,7 % der Theorie entspricht. Das kristalline Material wies einen Schmelzpunkt von 72 bis 76 ° C auf und zeigte im Massenspektrum einen Wert für m/e von 419 für das Molekülion.

Beispiel 37

Herstellung von 1-(4-Cyanophenoxy)-2-(4-cyclohexylphenoxy)-3,3,4,4,5,5-hexafluorcyclopenten-1

11,9 g (0,1 Mol) 4-Hydroxybenzonitril und 10,1 g Triethylamin in 50 ml Dimethylformamid wurden bei 5 ° C zu 21,2 g Perfluorcyclopenten in 15 Minuten zugetropft. Nach 2 Stunden Rühren bei Raumtemperatur wurden 19,8 g (0,1 Mol) festes Natrium-4-cyclohexylphenolat zum Reaktionsgemisch gegeben und weitere 16 Stunden bei 80 ° C gerührt. Das Reaktionsgemisch wurde anschließend in 100 ml Wasser eingetragen, die organische Phase in 100 ml Methylenchlorid aufgenommen, mit 50 ml 5 gew.-%iger Salzsäure, dann mit 50 ml 10 gew.-%iger Natronlauge und anschließend mit 100 ml Wasser gewaschen, über Natriumsulfat getrocknet und am Rotationsverdampfer zur Trockene eingedampft. Die Rohausbeute betrug 36,0 g. Das Produkt wurde aus Petrolether umkristallisiert und so 27 g kristallines Material mit einem Schmelzpunkt von 99 bis 103 ° C erhalten, was 58 % der Theorie entspricht. Das Massenspektrum zeigte einen Wert für m/e von 467 für das Molekülion.

Beispiel 38

Herstellung von [1,2-Bis-(4-carboxylphenoxy)-3,3,4,4,5,5-hexafluorcyclopenten-1]-Dinatriumsalz

190,4 g (0,4 Mol) des Produktes aus Beispiel 16 wurden in einem Gemisch aus 500 ml Dioxan und 100 ml Wasser gelöst und nach Zugabe von 3,2 g gepulvertem Natriumhydroxid 30 Min. am Rückfluß gekocht. Das Reaktionsgemisch wurde anschließend am Rotationsverdampfer zur Trockene eingedampft. Die Ausbeute an Produkt betrug 195 g, entsprechend 99 % der Theorie. Das Produkt wies einen Schmelzpunkt von über 280 ° C auf. Im Massenspektrum wurde eine Wert für m/e von 492 für das Molekülion gefunden.

Beispiel 39

Herstellung von 1,2-Bis-[4-(pentyloxycarbonyl)-phenoxy]-3,3,4,4,5,5-hexafluorcyclopenten-1

Zu 41,6 g p-Hydroxybenzoesäurepentylester (0,2 Mol) und 20,2 g Triethylamin (0,2 Mol) in 200 ml Methylenchlorid wurden bei 0 bis 5°C 21,2 g (0,1 Mol) Perfluorcyclopenten innerhalb von 10 Minuten zugetropft. Nach 16-stündigem Rühren bei Raumtemperatur wurde das Reaktionsgemisch nacheinander mit 100 ml Wasser, 100 ml 5 gew.-%iger Salzsäure und nochmals 100 ml Wasser gewaschen, anschließend getrocknet, dann das Methylenchlorid am Rotationsverdampfer abgezogen und das verbleibende Öl im Vakuum destilliert. Mit einem Siedepunkt von 146 bis 160°C bei 0,02 mbar wurden 30 g Produkt erhalten, was 51 % der Theorie entspricht. Das Massenspektrum ergab einen Wert von m/e von 588 für das Molekülion. Im $^{19}F$-Kernresonanzspektrum traten Banden bei $\delta$ = -36,0 ppm und bei $\delta$ = -51,5 ppm auf, gemessen gegen Trifluoressigsäure als externen Standard.

Beispiel 40

Herstellung von 1,2-Bis-[4-(chlorocarbonyl)phenoxy]-3,3,4,4,5,5-hexafluorcyclopenten-1

112 g (0,25 Mol) des Produktes aus Beispiel 20 wurden mit 150 g Thionylchlorid 16 Stunden am Rückfluß gerührt. Anschließend wurde überschüssiges Thionylchlorid bei Normaldruck und dann im Wasserstrahlvakuum abdestilliert. Der Rückstand wurde aus Ligroin umkristallisiert. Es wurden 84 g kristallines Produkt erhalten, das entspricht 69,3 % der Theorie. Das Produkt wies einen Schmelzpunkt von 82 bis 85°C auf. Im Massenspektrum wurde für m/e ein Wert von 484 ($^{35}Cl$) für das Molekülion bestimmt.

Beispiel 41

Herstellung von 1,2-Bis-(3,4-dicarboxylphenoxy)-3,3,4,4,5,5-hexafluorcyclopenten-1

In 200 ml Dimethylformamid wurden 36,4 g (0,2 Mol) 4-Hydroxyphthalsäure gelöst und bei 5°C mit 60,6 g (0,6 Mol) Triethylamin versetzt. Innerhalb 15 Minuten wurden 21,2 g (0,1 Mol) Perfluorcyclopenten zugetropft und 18 Stunden bei 50°C gerührt. Anschließend wurden 20 ml Methylenchlorid zugesetzt, das Reaktionsgemisch mit 200 ml Wasser, danach 200 ml 10 gew.-%iger Salzsäure und danach nochmals mit 200 ml Wasser gewaschen und getrocknet. Nach Abziehen des Lösungsmittels verblieben 23 g eines öligen Feststoffs, der aus heißem, mit wenig Salzsäure angesäuertem Wasser umkristallisiert wurde. Es wurden 17 g eines weißen Feststoffes mit einem Schmelzpunkt von 110 bis 115°C erhalten, was 31,7 % der Theorie entspricht. Aus dem Waschwasser konnten nach Ansäuern und Einengen weitere 25 g Rohprodukt gewonnen werden. Das umkristallisierte Material zeigte im $^{19}F$-Kernresonanzspektrum Banden bei $\delta$ = -35,7 ppm und bei $\delta$ = -51,4 ppm, gemessen gegen Trifluoressigsäure als externen Standard.

Beispiel 42

Herstellung von 1,2-Bis-(4-sulfophenoxy)-3,3,4,4,5,5-hexafluorcyclopenten-1

In 200 ml Dimethylformamid wurden 43,2 g (0,3 Mol) 4-Hydroxybenzolsulfonsäure (als 65 %ige wäßrige Lösung) und 60,6 g (0,6 Mol) Triethylamin bei 5°C vorgelegt. Innerhalb von 15 Minuten wurden 31,8 g (0,15 Mol) Perfluorcyclopenten zugetropft und 17 Stunden bei 60°C nachgerührt. Anschließend wurden 200 ml Methylenchlorid zugesetzt, das Reaktionsgemisch mit 200 ml Wasser, danach 200 ml 10 gew.-%iger Salzsäure und danach nochmals mit 200 ml Wasser gewaschen und getrocknet. Nach Abziehen des Lösungsmittels am Rotationsverdampfer verblieben 47,5 g eines ölig-kristallinen Rückstandes, der aus Eisessig umkristallisiert wurde. Es wurden 27,5 g kristallines Produkt mit einem Schmelzpunkt von 251 bis 255°C erhalten, was 35,3 % der Theorie entspricht. Aus dem Waschwasser wurden nach Ausäuern und Einengen weitere 35 g Rohprodukt isoliert. Das Massenspektrum des umkristallisierten Materials zeigte einen Wert von m/e von 520 für das Molekülion.

Beispiel 43

Herstellung von 1,2-Bis-[(4-benzyloxy)phenoxy]-3,3,4,4,5,5-hexafluorcyclopenten-1

In 500 ml Dimethylformamid wurden 200 g Hydrochinonmonobenzylether (1 Mol) mit 56,1 g gepulvertem Kaliumhydroxid bei 5 bis 10 °C 30 Minuten verrührt. Anschließend wurden aus einem gekühlten Tropftrichter innerhalb von 60 Min. 106 g (0,5 Mol) Perfluorcyclopenten unter Rühren zugetropft. Das Reaktionsgemisch wurde langsam auf Reaktionstemperatur gebracht und dann noch 16 Stunden bei 45 °C gerührt. Danach wurde der Ansatz in 1000 ml Wasser eingerührt, die organische Phase abgetrennt und mit Toluol aufgenommen. Das Toluol wurde zur Entfernung von Restwasser azeotrop abdestilliert. Es verblieben 232 g eines teilkristallinen Öls, was 81,4 % der Theorie entspricht. Das Massenspektrum zeigte für m/e einen Wert von 572 für das Molekülion an. Im $^{19}$F-NMR-Spektrum traten charakteristische Banden bei $\delta$ = -35,9 ppm und $\delta$ = -51,7 ppm auf, gemessen gegen Trifluoressigsäure als externen Standard.

Beispiel 44

Herstellung von 1,2-Bis-(4-hydroxyphenoxy)-3,3,4,4,5,5-hexafluorcyclopenten-1

a) In 300 ml Tetrahydrofuran wurden 143 g (0,25 Mol) des Produkts aus Beispiel 31 mit 5 g eines Palladium-auf-Kohle-Katalysators (Palladiumgehalt: 5 Gew.-%) bei einem Wasserstoffdruck von 5 bis 8 bar und einer Temperatur von 30 bis 40 °C 8 Stunden lang hydrogenolytisch gespalten. Das Reaktionsgemisch wurde anschließend filtriert und das Filtrat am Rotationsverdampfer zur Trockene eingedampft. Es wurden 96 g teilkristallines Produkt erhalten, was 98 % der Theorie entspricht. Nach Umfüllen aus Ethanol/Wasser (1:1) wurde aus Toluol umkristallisiert. Es wurden 83 g kristallines Material mit einem Schmelzpunkt von 107 bis 109 °C gewonnen, was 85 % der Theorie entspricht. Das Massenspektrum zeigte für m/e einen Wert von 392 für das Molekülion. Im $^{19}$F-Kernresonanzspektrum traten charakteristische Banden bei $\delta$ = -36,4 ppm und bei $\delta$ = -51,8 ppm auf, gemessen gegen Trifluoressigsäure als externen Standard.

b) In 200 ml Methanol wurden 100 g (1,0 Mol) Hydrochinon in der Wärme gelöst und mit 180 g 30 gew.-%iger Natriummethylatlösung in das Mononatriumsalz überführt. 132 g (1,0 Mol) des Natriumsalzes wurden anschließend portionsweise bei 5 °C zu einer Mischung von 250 ml Dimethylformamid und 106 g (0,5 Mol) Perfluorcyclopenten zugegeben. Nach 19 Stunden Rühren bei Raumtemperatur wurde der Ansatz in 500 ml Wasser gegeben, die organische Phase nach Zugabe von 1000 ml Methylenchlorid abgetrennt und mit 500 ml Wasser nachgewaschen. Nach dem Trocknen wurde das Lösungsmittel am Rotationsverdampfer abgezogen. Es verblieben 177 g eines teilkristallinen, öligen Materials. Zur weiteren Reinigung wurde in 200 ml Ethanol gelöst und durch Zugabe von 100 ml Wasser wieder ausgefällt. Es wurden 143 g eines leicht öligen Produktes erhalten, was 73 % der Theorie entspricht. Die spektroskopischen Daten (Massenspektrum und $^{19}$F-Kernresonanzspektrum) stimmten mit dem Produkt aus Beispiel 44a) überein.

Beispiel 45

Herstellung von 1,2-Bis-{4-[2-(4-hydroxyphenyl)prop-2-yl]phenoxy}-3,3,4,4,5,5-hexafluorcyclopenten-1

45,6 g Bisphenol-A (0,2 Mol) wurden in 100 ml Methanol unter Erwärmen gelöst und mit 36 g einer 30 gew.-%igen Natriummethylatlösung versetzt. Das Gemisch wurde am Rotationsverdampfer bis zur Trockene eingedampft, wobei 61 g Mononatrium-Bisphenol-A verblieben. Diese wurden in 100 ml Dimethylformamid gelöst, bei 5 °C innerhalb von 60 Minuten mit 21,2 g Perfluorcyclopenten (0,1 Mol) versetzt, dann auf Raumtemperatur erwärmt und 18 Stunden bei Raumtemperatur gerührt. Danach wurde für 2 Stunden auf 70 °C erwärmt. Anschließend wurde der Ansatz in 200 ml Wasser eingerührt und die organische Phase abgetrennt. Es wurde in Methylenchlorid aufgenommen, die Lösung mit 100 ml Wasser, danach 50 ml 5 gew.-%iger Salzsäure und danach nochmals mit 100 ml Wasser gewaschen und anschließend getrocknet. Nach dem Abziehen des Lösungsmittels am Rotationsverdampfer und Trocknen im Vakuum einer Ölpumpe bei 100 °C verblieben 53 g eines glasartigen, transparenten Rückstandes, was 90,7 % der Theorie entspricht. Das $^{19}$F-Kernresonanzspektrum zeigte charakteristische Banden bei $\delta$ = -36,1 ppm und bei $\delta$ = -51,5 ppm, gemessen gegen Trifluoressigsäure als externen Standard.

Beispiel 46

Herstellung von 1,2-Bis-(4-nitrophenoxy)-3,3,4,4,5,5,6,6-octafluorcyclohexen-1

26,2 g (0,1 Mol) Dekafluorcyclohexen wurden bei 0 bis 5 °C im Verlauf von 15 Minuten in eine Mischung aus 27,8 g (0,2 Mol) 4-Nitrophenol und 20,2 g (0,2 Mol) Triethylamin gelöst in 200 ml Methylenchlorid zugefügt. Die Mischung wurde auf Raumtemperatur gebracht und anschließend für 16 Stunden gerührt. Zur Aufarbeitung wurde das Reaktionsgemisch nacheinander zweimal jeweils mit 100 ml Wasser, einmal mit 100 ml 5 gew.-%iger wäßriger Salzsäure und nochmals mit 100 ml Wasser gewaschen, anschließend über Natriumsulfat getrocknet und das Methylenchlorid bis zur Trockene an einem Rotationsverdampfer entfernt. Es wurden 45 g Rohprodukt (= 90 % der Theorie) mit einem Schmelzpunkt von 150 - 165 °C erhalten. Nach der Kristallisation aus Methylenchlorid betrug der Schmelzpunkt des Produktes 158 - 166 °C. Das Massenspektrum ergab einen Wert für m/e von 500 für das Molekülion. Das $^{19}F$-NMR-Spektrum zeigte Banden bei $\delta$ = -37,1 ppm und -54,8 ppm, gemessen gegen Trifluoressigsäure als externen Standard.

**Patentansprüche**

**1.** Fluorierte Bis-aryloxy-substituierte Alkene der Formel (I)

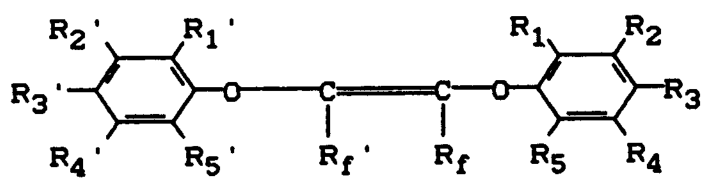

(I),

in der

$R_f$ und $R_f'$ unabhängig voneinander für einen $C_1$- bis $C_6$-Perfluoralkylrest oder $R_f$ und $R_f'$ gemeinsam für einen $C_2$- bis $C_4$-Perfluoralkylenrest und

$R_1$ bis $R_5$ und $R_1'$ bis $R_5'$ unabhängig voneinander für Wasserstoff, für $C_1$- bis $C_{12}$-Alkyl, $C_2$-bis $C_6$-Alkenyl und/oder $C_2$- bis $C_6$-Alkinyl, die gegebenenfalls mit OCN-, $R_6O$-, $H_2N$-, $R_6SO_3^-$und/oder $R_6OOC$-Gruppen (mit $R_6$ = Wasserstoff oder gegebenenfalls substituiertem $C_1$- bis $C_6$-Alkyl oder gegebenenfalls substituiertem $C_5$-bis $C_7$-Cycloalkyl) substituiert sind und wobei Alkinylgruppen auch Silyl-Substituenten enthalten können, für $C_1$-bis $C_4$-Halogenalkyl, für $C_1$- bis $C_4$-Epoxyalkyl, für $C_1$- bis $C_4$-Hydroxyalkyl, für gegebenenfalls substituiertes Phenyl, für $-OR_7$ (mit $R_7$ = Wasserstoff, $C_1$- bis $C_6$-Alkyl, $C_1$-bis $C_4$-Hydroxyalkyl, $C_1$- bis $C_4$-Epoxyalkyl, $-(CH_2)_m-[-O-(-CH_2)_m-]-_nOH$ mit m = 1 bis 4 und n = 1 bis 8, gegebenenfalls substituiertem Phenyl oder gegebenenfalls substituiertem Benzyl), für Halogen, für $NO_2$, für $NH_2$, für NCO, für CN, für $SO_3X$ (mit X = Wasserstoff, Natrium oder Kalium), für S-$C_1$- bis $C_6$-Alkyl, für $SO_2$Halogen, für gegebenenfalls substituiertes $C_5$- bis $C_7$-Cycloalkyl, für Carbonyl-$R_8$ (mit $R_8$ = Wasserstoff, gegebenenfalls substituiertem $C_1$- bis $C_6$-Alkyl, gegebenenfalls substituiertem $C_5$- bis $C_7$-Cycloalkyl, Halogen, gegebenenfalls substituiertem $C_1$-bis $C_6$-Alkoxy, gegebenenfalls substituiertem $C_5$-bis $C_7$-Cycloalkoxy oder OY mit Y = Wasserstoff, Natrium oder Kalium) oder für

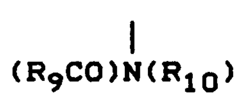

(mit $R_9$ = $C_1$- bis $C_6$-Alkyl, $C_1$-bis $C_4$-Halogenalkyl, gegebenenfalls substituiertem Phenyl oder Wasserstoff und mit $R_{10}$ = Wasserstoff

oder $C_1$- bis $C_4$-Alkyl) stehen,
wobei die Verbindung ausgenommen ist, bei der $R_1$ bis $R_5$ und $R_1'$ bis $R_5'$ für Wasserstoff und $R_f$ und $R_f'$ gemeinsam für $-(-CF_2-)-_3$ stehen.

2. Verbindungen nach Anspruch 1, dadurch gekennzeichnet, daß $R_f$ und $R_{f'}$ gleich sind und für $CF_3$ stehen oder $R_f$ und $R_{f'}$ gemeinsam eine $-CF_2-CF_2-$ oder eine $-CF_2-CF_2-CF_2-$ Gruppe bilden.

3. Verbindungen nach Ansprüchen 1 und 2, dadurch gekennzeichnet, daß jeweils $R_1$ und $R_{1'}$ und unabhängig davon jeweils $R_5$ und $R_{5'}$ gleich sind und für Wasserstoff, $C_1$- bis $C_4$-Alkyl, $C_1$- bis $C_4$-Halogenalkyl, $NO_2$, NCO, Fluor oder Chlor stehen,
jeweils $R_2$ und $R_{2'}$ und unabhängig davon jeweils $R_4$ und $R_{4'}$ gleich sind und für Wasserstoff, $C_1$- bis $C_4$-Alkyl, COOH, Fluor, Chlor oder $C_1$- bis $C_4$-Halogenalkyl und
$R_3$ und $R_{3'}$ unabhängig voneinander für Wasserstoff, für $C_1$- bis $C_{12}$-Alkyl, $C_2$- bis $C_6$-Alkenyl und/oder $C_2$- bis $C_6$-Alkinyl, die gegebenenfalls mit OCN-, $R_6O$-, $H_2N$-, $R_6SO_3^-$ und/oder $R_6OOC$-Gruppen (mit $R_6$ = Wasserstoff oder gegebenenfalls substituiertem $C_1$- bis $C_6$-Alkyl oder gegebenenfalls substituiertem $C_5$- bis $C_7$-Cycloalkyl) substituiert sind und wobei Alkinylgruppen auch Silyl-Substituenten enthalten können, für $C_1$- bis $C_4$-Halogenalkyl, für $C_1$-bis $C_4$-Epoxyalkyl, für $C_1$- bis $C_4$-Hydroxyalkyl, für gegebenenfalls substituiertes Phenyl, für $-OR_7$ (mit $R_7$ = Wasserstoff, $C_1$- bis $C_6$-Alkyl, $C_1$-bis $C_4$-Hydroxyalkyl, $C_1$- bis $C_4$-Epoxyalkyl, $-(-CH_2)_m-[-O-(-CH_2)_m-]-_nOH$ mit m = 1 bis 4 und n = 1 bis 8, gegebenenfalls substituiertem Phenyl oder gegebenenfalls substituiertem Benzyl), für Halogen, für $NO_2$, für $NH_2$, für NCO, für CN, für $SO_3X$ (mit X = Wasserstoff, Natrium oder Kalium), für S-$C_1$-bis $C_6$-Alkyl, für $SO_2$Halogen, für gegebenenfalls substituiertes $C_5$- bis $C_7$-Cycloalkyl, für Carbonyl-$R_8$ (mit $R_8$ = Wasserstoff, gegebenenfalls substituiertem $C_1$- bis $C_6$-Alkyl, gegebenenfalls substituiertem $C_5$- bis $C_7$-Cycloalkyl, Halogen, gegebenenfalls substituiertem $C_1$-bis $C_6$-Alkoxy, gegebenenfalls substituiertem $C_5$- bis $C_7$-Cycloalkoxy oder OY mit Y = Wasserstoff, Natrium oder Kalium) oder für

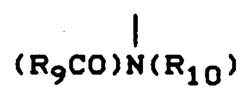

(mit $R_9$ = $C_1$ bis $C_6$-Alkyl, $C_1$- bis $C_4$-Halogenalkyl, gegebenenfalls substituiertem Phenyl oder Wasserstoff und mit $R_{10}$ = Wasserstoff oder $C_1$- bis $C_4$-Alkyl) stehen.

4. Verbindungen nach Ansprüchen 1 bis 3, dadurch gekennzeichnet, daß $R_f$ und $R_f'$ jeweils für $CF_3$ stehen oder $R_f$ und $R_f'$ gemeinsam eine $-CF_2-CF_2-$ oder eine $-CF_2-CF_2-CF_2$-Gruppe bedeuten, $R_1$, $R_2$, $R_4$, $R_5$, $R_1'$, $R_2'$, $R_4'$ und $R_5'$ für Wasserstoff stehen und $R_3$ und $R_3'$ gleich sind une eine in Anspruch 3 angegebene, von Wasserstoff verschiedenen Bedeutung haben.

5. Verfahren zur Herstellung von fluorierten Bis-aryloxy-substituierten Alkenen der Formel (I), dadurch gekennzeichnet, daß man ein Phenol und/oder Phenolat der Formel (II)

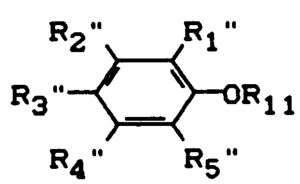

(II),

in der

$R_1''$ bis $R_5''$ unabhängig voneinander für Wasserstoff, für $C_1$- bis $C_{12}$-Alkyl, $C_2$- bis $C_6$-Alkenyl und/oder $C_2$- bis $C_6$-Alkinyl, die gegebenenfalls mit $R_6'O$-, $(R_6')_2N$-, $R_6'SO_3$- und/oder $R_6'OOC$-Gruppen (mit $R_6'$ = gegebenenfalls substituiertem $C_1$- bis $C_6$-Alkyl oder gegebenenfalls substituiertem $C_5$- bis $C_7$-Cycloalkyl) substituiert sind und wobei Alkinylgruppen auch Trimethylsilyl-Substituenten enthalten können, für $C_1$-bis $C_4$-Halogenalkyl, für gegebenenfalls substituiertes Phenyl, für $OR_7'$ (mit $R_7'$ = $C_1$-

bis $C_6$-Alkyl, gegebenenfalls substituiertem Phenyl oder gegebenenfalls substituiertem Benzyl), für Halogen, für $NO_2$, für $NH_2$ mit nicht aciden H-Atomen, für CN, für $SO_3X'$ (mit $X'$ = Natrium oder Kalium) für S-$C_1$- bis $C_6$-Alkyl, für gegebenenfalls substituiertes $C_5$-bis $C_7$-Cycloalkyl, für Carbonyl-$R_8'$ (mit $R_8'$ = gegebenenfalls substituiertem $C_1$-bis $C_6$-Alkyl, $C_1$- bis $C_6$-Alkoxy, $C_5$-bis $C_7$-Cycloalkyl oder $C_5$-bis $C_7$-Cycloalkoxy oder $OY'$ mit $Y'$ = Natrium oder Kalium) oder für

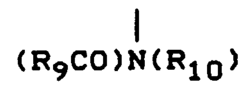

(mit $R_9$ = $C_1$- bis $C_6$-Alkyl, $C_1$- bis $C_4$-Halogenalkyl, gegebenenfalls substituiertem Phenyl oder Wasserstoff und mit $R_{10}$ = Wasserstoff oder $C_1$-bis $C_4$-Alkyl) und

$R_{11}$ für Wasserstoff, einen Tri-$C_1$-$C_4$-alkylsilylrest oder ein Alkalimetall stehen

mit einem Perhalogenalken der Formel (III)

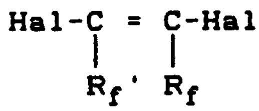

(III),

in der

$R_f$ und $R_f'$ die bei Formel (I) angegebene Bedeutung haben und

Hal für Fluor, Chlor, Brom und/oder Iod stehen,

in basischem Milieu und in Gegenwart eines Lösungsmittels umsetzt und gegebenenfalls anschließend eine Derivatisierung und/oder Umderivatisierung durchführt, wobei die Umsetzung von unsubstituiertem Phenol und unsubstituierten Phenolaten (Formel (II), $R_1''$ bis $R_5''$ = H, $R_{11}$ = H oder Alkalimetall) mit Octafluorcyclopenten (Formel (III)), $R_f$ und $R_f'$ = gemeinsam $(CF_2)_3$, Hal (= F) ausgenommen ist.

6. Verfahren nach Anspruch 5, dadurch gekennzeichnet, daß man in Abwesenheit von aciden Wasserstoffatomen 1,7 bis 2,3 Mol Base pro Mol der Verbindung der Formel (III) einsetzt.

7. Verfahren nach Ansprüchen 5 und 6, dadurch gekennzeichnet, daß das Molverhältnis der jeweils eingesetzten Verbindung der Formel (II) zu der jeweils eingesetzten Verbindung der Formel (III) 1,9:1 bis 3:1 beträgt.

8. Verwendung von fluorierten Bis-aryloxy-substituierten Alkenen der Formel (I) als Elektroisoliermittel.

## Claims

1. Fluorinated bisaryloxy-substituted alkenes of the formula (I)

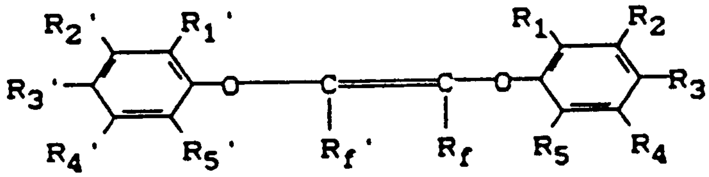

(I),

in which

$R_f$ and $R_f'$ independently of one another represent a $C_1$- to $C_6$-perfluoroalkyl radical or $R_f$ and $R_f'$ together represents a $C_2$- to $C_4$-perfluoroalkylene radical and

$R_1$ to $R_5$ and $R_1'$ to $R_5'$ independently of one another represent hydrogen, $C_1$- to $C_{12}$-alkyl, $C_2$- to $C_6$-alkenyl and/or $C_2$- to $C_6$-alkinyl, which are optionally substituted with OCN-, $R_6O$-, $H_2N$-, $R_6SO_3$- and/or $R_6OOC$- groups (with $R_6$ =

hydrogen or optionally substituted $C_1$- to $C_6$-alkyl or optionally substituted $C_5$- to $C_7$-cycloalkyl) and where alkinyl groups may also contain silyl substituents, $C_1$- to $C_4$-halogenoalkyl,$C_1$- to $C_4$-epoxyalkyl, $C_1$- to $C_4$-hydroxyalkyl, optionally substituted phenyl, $-OR_7$ (with $R_7$ = hydrogen, $C_1$- to $C_6$-alkyl, $C_1$- to $C_4$-hydroxyalkyl, $C_1$- to $C_4$-epoxyalkyl, $-(-CH_2)_m-[-O-(-CH_2)_m-]-_nOH$ with m = 1 to 4 and n = 1 to 8, optionally substituted phenyl or optionally substituted benzyl), halogen, $NO_2$, $NH_2$, NCO, CN, $SO_3X$ (with X = hydrogen, sodium or potassium), S-$C_1$- to $C_6$-alkyl, $SO_2$halogen, optionally substituted $C_5$- to $C_7$-cycloalkyl, carbonyl-$R_8$ (with $R_8$ = hydrogen, optionally substituted $C_1$- to $C_6$-alkyl, optionally substituted $C_5$- to $C_7$-cycloalkyl, halogen, optionally substituted $C_1$- to $C_6$-alkoxy, optionally substituted $C_5$- to $C_7$-cycloalkoxy or OY with Y = hydrogen, sodium or potassium) or

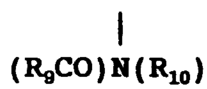

(with $R_9$ = $C_1$- to $C_6$-alkyl, $C_1$- to $C_4$-halogenoalkyl, optionally substituted phenyl or hydrogen and with $R_{10}$ = hydrogen or $C_1$- to $C_4$-alkyl), where the compound is excluded in which $R_1$ to $R_5$ and $R_1$' to $R_5$' represent hydrogen and $R_f$ and $R_f$' together represent $-(-CF_2-)-_3$.

**2.** Compounds according to Claim 1, characterised in that $R_f$ and $R_f$' are identical and represent $CF_3$ or $R_f$ and $R_f$' together form a $-CF_2-CF_2-$ or a $-CF_2-CF_2-CF_2-$ group.

**3.** Compounds according to Claims 1 and 2, characterised in that in each case $R_1$ and $R_1$' and independently thereof in each case $R_5$ and $R_5$' are identical and represent hydrogen, $C_1$- to $C_4$-alkyl, $C_1$- to $C_4$-halogenoalkyl, $NO_2$, NCO, fluorine or chlorine and in each case $R_2$ and $R_2$' and independently thereof in each case $R_4$ and $R_4$' are identical and represent hydrogen, $C_1$- to $C_4$-alkyl, COOH, fluorine, chlorine or $C_1$- to $C_4$-halogenoalkyl and
$R_3$ and $R_3$' independently of one another represent hydrogen, $C_1$- to $C_{12}$-alkyl, $C_2$- to $C_6$-alkenyl and/or $C_2$- to $C_6$-alkinyl, which are optionally substituted by OCN-, $R_6$O-, $H_2N$-, $R_6SO_3$- and/or $R_6$OOC- groups (with $R_6$ = hydrogen or optionally substituted $C_1$- to $C_6$-alkyl or optionally substituted $C_5$- to $C_7$-cycloalkyl) and where alkinyl groups may also contain silyl substituents, $C_1$- to $C_4$-halogenoalkyl, $C_1$- to $C_4$-epoxyalkyl, $C_1$- to $C_4$-hydroxyalkyl, optionally substituted phenyl, $-OR_7$ (with $R_7$ = hydrogen, $C_1$- to $C_6$-alkyl, $C_1$- to $C_4$-hydroxyalkyl, $C_1$- to $C_4$-epoxyalkyl, $-(-CH_2)_m-[-O-(CH_2)_m-]-_nOH$ with m = 1 to 4 and n = 1 to 8, optionally substituted phenyl or optionally substituted benzyl), halogen, $NO_2$, $NH_2$, NCO, CN, $SO_3X$ (with X = hydrogen, sodium or potassium), S-$C_1$- to $C_6$-alkyl, $SO_2$-halogen, optionally substituted $C_5$- to $C_7$-cycloalkyl, carbonyl-$R_8$ (with $R_8$ = hydrogen, optionally substituted $C_1$- to $C_6$-alkyl, optionally substituted $C_5$- to $C_7$-cycloalkyl, halogen, optionally substituted $C_1$- to $C_6$-alkoxy, optionally substituted $C_5$- to $C_7$-cycloalkoxy or OY with Y = hydrogen, sodium or potassium) or

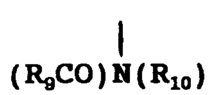

(with $R_9$ = $C_1$- to $C_6$-alkyl, $C_1$- to $C_4$-halogenoalkyl, optionally substituted phenyl or hydrogen and with $R_{10}$ = hydrogen or $C_1$- to $C_4$-alkyl).

**4.** Compounds according to Claims 1 to 3, characterised in that $R_f$ and $R_f$' in each case represent $CF_3$ or $R_f$ and $R_f$' together denote a $-CF_2-CF_2-$ or a $-CF_2-CF_2-CF_2-$ group, $R_1$, $R_2$, $R_4$, $R_5$, $R_1$', $R_2$', $R_4$' and $R_5$' represent hydrogen and $R_3$ and $R_3$' are identical and have a meaning other than hydrogen indicated in Claim 3.

**5.** Process for the preparation of fluorinated bis-aryloxy-substituted alkenes of the formula (I), characterised in that a phenol and/or phenoxide of the formula (II)

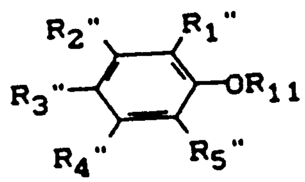

(II)

in which

$R_1$" to $R_5$" independently of one another represent hydrogen, $C_1$- to $C_{12}$-alkyl, $C_2$- to $C_6$-alkenyl and/or $C_2$- to $C_6$-alkinyl which are optionally substituted by $R_6$'O-, $(R_6')_2N$-,$R_6$'$SO_3$- and/or $R_6$'OOC- groups (with $R_6$' = optionally substituted $C_1$- to $C_6$-alkyl or optionally substituted $C_5$- to $C_7$-cycloalkyl) and where alkinyl groups may also contain trimethylsilyl substituents, $C_1$- to $C_4$-halogenoalkyl, optionally substituted phenyl, $OR_7$' (with $R_7$' = $C_1$- to $C_6$-alkyl, optionally substituted phenyl or optionally substituted benzyl), halogen, $NO_2$, $NH_2$ with non-acidic H atoms, CN, $SO_3X$' (with X' = sodium or potassium) S-$C_1$- to $C_6$-alkyl, optionally substituted $C_5$- to $C_7$-cycloalkyl, carbonyl-$R_8$' (with $R_8$' = optionally substituted $C_1$- to $C_6$-alkyl, $C_1$- to $C_6$-alkoxy, $C_5$- to $C_7$-cycloalkyl or $C_5$- to $C_7$-cycloalkoxy or OY' with Y' = sodium or potassium) or

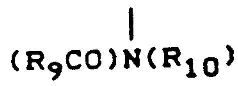

(with $R_9$ = $C_1$- to $C_6$-alkyl, $C_1$- to $C_4$-halogenoalkyl, optionally substituted phenyl or hydrogen and with $R_{10}$ = hydrogen or $C_1$- to $C_4$-alkyl) and

$R_{11}$ represents hydrogen, a tri-$C_1$-$C_4$-alkylsilyl radical or an alkali metal

are reacted with a perhaloalkene of the formula (III)

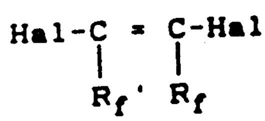

(III)

in which

$R_f$ and $R_f$' have the meaning indicated in formula (I) and

Hal represents fluorine, chlorine, bromine and/or iodine,

in basic medium and in the presence of a solvent and if appropriate a derivatisation and/or rederivatisation are subsequently carried out, where the reaction of unsubstituted phenol and unsubstituted phenoxides (formula (II), $R_1$" to $R_5$" = H, $R_{11}$ = H or alkali metal) with octafluorocyclopentene (formula (III), $R_f$ and $R_f$' together = $(CF_2)_3$, Hal = F) is excluded.

6. Process according to Claim 5, characterised in that, in the absence of acidic hydrogen atoms, 1.7 to 2.3 moles of base are employed per mole of the compound of the formula (III).

7. Process according to Claims 5 and 6, characterised in that the molar ratio of the compound of the formula (II) employed in each case to the compound of the formula (III) employed in each case is 1.9:1 to 3:1.

8. Use of fluorinated bisaryloxy-substituted alkenes of the formula (I) as electrical insulating agents.

## Revendications

1. Alcènes fluorés portant deux substituants aryloxy, de formule (I)

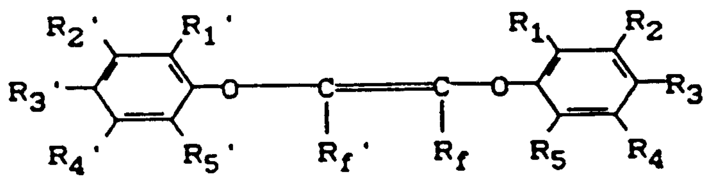

(I),

dans laquelle

$R_f$ et $R_f'$ représentent indépendamment l'un de l'autre un reste perfluoralkyle en $C_1$ à $C_6$ ou bien $R_f$ et $R_f'$ forment conjointement un reste perfluoralkylène en $C_2$ à $C_4$ et

$R_1$ à $R_5$ et $R_1'$ à $R_5'$ représentent indépendamment l'un de l'autre de l'hydrogène, un groupe alkyle en $C_1$ à $C_{12}$, un groupe alcényle en $C_2$ à $C_6$ et/ou un groupe alcynyle en $C_2$ à $C_6$ qui sont éventuellement substitués avec des groupes OCN, $R_6O$, $H_2N$, $R_6SO_3$- et/ou $R_6OOC$ (avec $R_6$ = hydrogène ou groupe alkyle en $C_1$ à $C_6$ éventuellement substitué ou groupe cycloalkyle en $C_5$ à $C_7$ éventuellement substitué) et les groupes alcynyle peuvent aussi contenir des substituants silyle, un groupe halogénalkyle en $C_1$ à $C_4$, un groupe époxyalkyle en $C_1$ à $C_4$, un groupe hydroxyalkyle en $C_1$ à $C_4$, un groupe phényle éventuellement substitué, un groupe $-OR_7$ (avec $R_7$ = hydrogène, ou groupe alkyle en $C_1$ à $C_6$, hydroxyalkyle en $C_1$ à $C_4$, époxyalkyle en $C_1$ à $C_4$, $-(-CH_2)_m-[-O-(-CH_2)_m-]-_nOH$ avec m = 1 à 4 et n = 1 à 8, phényle éventuellement substitué ou benzyle éventuellement substitué), un halogène, un groupe $NO_2$, un groupe $NH_2$, un groupe NCO, un groupe CN, un groupe $SO_3X$ (avec X = hydrogène, sodium ou potassium), un groupe S-(alkyle en $C_1$ à $C_6$), $SO_2$halogène, un groupe cycloalkyle en $C_5$ à $C_7$ éventuellement substitué, un groupe carbonyle $-R_8$ (avec $R_8$ = hydrogène, alkyle en $C_1$ à $C_6$ éventuellement substitué, cycloalkyle en $C_5$ à $C_7$ éventuellement substitué, halogène, alkoxy en $C_1$ à $C_6$ éventuellement substitué, cycloalkoxy en $C_5$ à $C_7$ éventuellement substitué ou OY avec Y = hydrogène, sodium ou potassium) ou un groupe

$$(R_9CO)\overset{|}{N}(R_{10})$$

(avec $R_9$ = alkyle en $C_1$ à $C_6$, halogénalkyle en à $C_1$ à $C_4$, phényle éventuellement substitué ou hydrogène et avec $R_{10}$ = hydrogène ou alkyle en $C_1$ à $C_4$), à l'exception du composé dans lequel $R_1$ à $R_5$ et $R_1'$ à $R_5'$ représentent l'hydrogène et $R_f$ et $R_f'$ forment conjointement un groupe $-(-CF_2-)-_3$.

2. Composés suivant la revendication 1, caractérisés en ce que $R_f$ et $R_f'$ sont identiques et représentent des groupes $CF_3$, ou bien $R_f$ et $R_f'$ forment conjointement un groupe $-CF_2-CF_2$ ou un groupe $-CF_2-CF_2-CF_2-$.

3. Composés suviant les revendications 1 et 2, caractérisés en ce que $R_1$ et $R_1'$ et, indépendamment d'eux $R_5$ et $R_5'$ sont identiques et représentent chacun l'hydrogène, un groupe alkyle en $C_1$ à $C_4$, halogénalkyle en $C_1$ à $C_4$, $NO_2$, NCO, le fluor ou le chlore, $R_2$ et $R_2'$ et, indépendamment d'eux $R_4$ et $R_4'$ sont identiques et représentent chacun l'hydrogène, un groupe alkyle en $C_1$ à $C_4$, COOH, le fluor, le chlore ou un groupe halogénalkyle en $C_1$ à $C_4$ et $R_3$ et $R_3'$ représentent indépendamment l'un de l'autre l'hydrogène, un groupe alkyle en $C_1$ à $C_{12}$, un groupe alcényle en $C_2$ à $C_6$ et/ou un groupe alcynyle en $C_2$ à $C_6$ qui sont éventuellement substitués avec des groupes OCN, $R_6O$, $H_2N$, $R_6SO_3$-

et/ou $R_6OOC$ (avec $R_6$ = hydrogène ou alkyle en $C_1$ à $C_6$ éventuellement substitué ou cycloalkyle en $C_5$ à $C_7$ éventuellement substitué) et les groupes alcynyle peuvent aussi porter des substituants silyle, un groupe halogénalkyle en $C_1$ à $C_4$, un groupe époxyalkyle en $C_1$ à $C_4$, un groupe hydroxyalkyle en $C_1$ à $C_4$, un groupe hydroxyalkyle en $C_1$ à $C_4$, un groupe phényle éventuellement substitué, un groupe $-OR_7$ (avec $R_7$ = hydrogène, alkyle en $C_1$ à $C_6$, hydroxyalkyle en $C_1$ à $C_4$, époxyalkyle en $C_1$ à $C_4$, un groupe $-(-CH_2)_m-[-O-(-CH_2)_m-]-_nOH$ avec m = 1 à 4 et n = 1 à 8, un groupe phényle éventuellement substitué ou benzyle éventuellement substitué), un halogène, un groupe $NO_2$, $NH_2$, NCO, CN, $SO_3X$ (avec X = hydrogène, sodium ou potassium), un groupe S-(alkyle en $C_1$ à $C_6$), un groupe $SO_2$halogène, un groupe cycloalkyle en $C_5$ à $C_7$ éventuellement substitué, un groupe carbonyl-$R_8$ avec $R_8$ = hydrogène, alkyle en $C_1$ à $C_6$ éventuellement substitué, cycloalkyle en $C_5$ à $C_7$ éventuellement substitué, un halogène, un groupe alkoxy en $C_1$ à $C_6$ éventuellement substitué, cycloalkoxy en $C_5$ à $C_7$ éventuellement substitué ou un groupe OY, avec Y = hydrogène, sodium ou potassium) ou un groupe

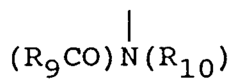

(avec $R_9$ = alkyle en $C_1$ à $C_6$, halogénalkyle en $C_1$ à $C_4$, phényle éventuellement substitué ou hydrogène et avec $R_{10}$ = hydrogène ou alkyle en $C_1$ à $C_4$).

**4.** Composés suivant les revendications 1 à 3, caractérisés en ce que $R_f$ et $R_f'$ représentent chacun $CF_3$ ou bien $R_f$ et $R_f'$ forment conjointement un groupe $-CF_2-CF_2-$ ou un groupe $-CF_2-CF_2-CF_2-$, $R_1$, $R_2$, $R_4$, $R_5$, $R_1'$, $R_2'$, $R_4'$ et $R_5'$ représentent l'hydrogène et $R_3$ et $R_3'$ sont identiques et ont une définition, différente de l'hydrogène, indiquée dans la revendication 3.

**5.** Procédé de production d'alcènes fluorés portant deux substituants aryloxy de formule (I), caractérisé en ce qu'on fait réagir un phénol et/ou un phénolate de formule (II)

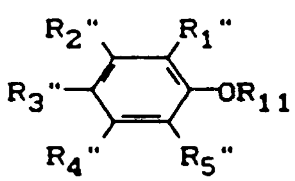

(II),

dans laquelle

$R_1''$ à $R_5''$ représentent, indépendamment les uns des autres, l'hydrogène, un groupe alkyle en $C_1$ à $C_{12}$, un groupe alcényle en $C_2$ à $C_6$ et/ou un groupe alcynyle en $C_2$ à $C_6$ qui sont éventuellement substitués avec des groupes $R_6'O$, $(R_6')_2N$, $R_6'SO_3-$ et/ou $R_6'OOC$ (avec $R_6'$ = groupe alkyle en $C_1$ à $C_6$ éventuellement substitué ou groupe cycloalkyle en $C_5$ à $C_7$ éventuellement substitué) et les groupes alcynyle peuvent aussi contenir des substituants triméthylsilyle, un groupe halogénalkyle en $C_1$ à $C_4$, un groupe phényle éventuellement substitué, un groupe $OR_7'$ (avec $R_7'$ = alkyle en $C_1$ à $C_6$, phényle éventuellement substitué ou benzyle éventuellement substitué), un halogène, un groupe $NO_2$, un groupe $NH_2$ avec des atomes d'hydrogène non acides, un groupe CN, un groupe $SO_3X'$ (avec X' = sodium ou potassium), un groupe S-(alkyle en $C_1$ à $C_6$), un groupe cycloalkyle en $C_5$ à $C_7$ éventuellement substitué, un groupe carbonyl-$R_8'$ (avec $R_8'$ = groupe alkyle en $C_1$ à $C_6$ éventuellement substitué, groupe alkoxy en $C_1$ à $C_6$, groupe cycloalkyle en $C_5$ à $C_7$ ou groupe cycloalkoxy en $C_5$ à $C_7$ ou groupe OY', avec Y' = sodium ou potassium) ou un groupe

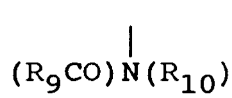

(avec $R_9$ = groupe alkyle en $C_1$ à $C_6$, halogénalkyle en $C_1$ à $C_4$, phényle éventuellement substitué ou hydrogène et avec $R_{10}$ = hydrogène ou alkyle en $C_1$ à $C_4$) et

$R_{11}$ est l'hydrogène, un reste tri(alkyle en $C_1$ à $C_4$)silyle ou un métal alcalin avec un alcène perhalogéné de formule (III)

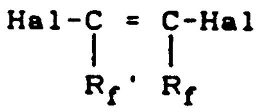 (III),

dans laquelle

$R_f$ et $R_f'$ ont la définition indiquée pour la formule (I) et

Hal représente le fluor, le chlore, le brome et/ou l'iode,

en milieu basique et en présence d'un solvant et on conduit ensuite le cas échéant une dérivatisation et/ou une transdérivatisation, la réaction de phénol non substitué et de phénolates non substitués (formule (II), $R_1''$ à $R_5''$ = H, $R_{11}$ = H ou métal alcalin) avec l'octafluorocyclopentène (formule III)), $R_f$ et $R_f'$ formant conjointement un groupe $(CF_2)_3$, Hal (= F) étant exclu.

**6.** Procédé suivant la revendication 5, caractérisé en ce qu'on utilise en l'absence d'atomes acides d'hydrogène 1,7 à 2,3 moles de base par mole de composé de formule (III).

**7.** Procédé suivant les revendications 5 et 6, caractérisé en ce que le rapport molaire du composé de formule (II) utilisé dans chaque cas au composé de formule (III) utilisé dans chaque cas a une valeur de 1,9:1 à 3:1.

**8.** Utilisation d'alcènes fluorés portant deux substituants aryloxy de formule (I) comme agents d'isolation électrique.